# EUROPÄISCHE PATENTANMELDUNG

(11) **EP 4 752 903 A1**
(43) Veröffentlichungstag der Anmeldung: **03.06.2026**
(21) Anmeldenummer: 24216645.2
(22) Anmeldetag: 29.11.2024
(51) Int. Cl.: G16H 50/20

(54) **VERFAHREN UND SYSTEME ZUM BESTIMMEN UND ÜBERWACHEN EINER BLUTBEZOGENEN, INDIVIDUELLEN ANAEROBEN SCHWELLE, VERFAHREN ZUM TRAINIEREN EINES ENTSPRECHENDEN DATENMODELLS, VORRICHTUNG, COMPUTERPROGRAMM, COMPUTERLESBARES MEDIUM**

(71) Anmelder: Ivoclar Vivadent AG, 9494 Schaan (LI)
(72) Erfinder: SKAWRAN, Alexander, 7320 Sargans (CH); GROSSE-HONEBRINK, Alexander, 9463 Oberriet (CH)
(74) Vertreter: Maiwald GmbH

(57) **Zusammenfassung**

Es wird ein Verfahren zum Bestimmen einer blutbezogenen, individuellen anaeroben Schwelle (ANS) eines Probanden (10) beschrieben. Hierzu werden erste Daten (D1) erhalten, welche eine Leistung des Probanden (10) beschreibende Leistungswerte (P), einen jeweils zugehörigen Erfassungszeitpunkt beschreibende Zeitwerte (T) und jeweils zugehörige Speichellaktatkonzentrationswerte (KS) umfassen. Die ersten Daten (D1) gehen über einen maximalen Speichellaktatkonzentrationswert (KS) hinaus. Basierend auf den ersten Daten (D1) und unter Nutzung eines Datenmodells (M) wird die blutbezogene, individuelle anaerobe Schwelle (ANS) erhalten. Ferner wird ein Verfahren zum Überwachen einer blutbezogenen, individuellen anaeroben Schwelle (ANS) eines Probanden (10) vorgestellt. Außerdem wird ein Verfahren zum Trainieren eines Datenmodells (M) zum Bestimmen einer blutbezogenen, individuellen anaeroben Schwelle (ANS) präsentiert. Zudem werden eine Vorrichtung (22) zur Datenverarbeitung, ein Computerprogramm (32) und ein computerlesbares Medium (30) erläutert. Auch werden ein System (16) zum Bestimmen einer blutbezogenen, individuellen anaeroben Schwelle (ANS) sowie ein System zum Überwachen einer blutbezogenen, individuellen anaeroben Schwelle (ANS) beschrieben.

## Beschreibung

Die Erfindung betrifft ein Verfahren zum Bestimmen einer blutbezogenen, individuellen anaeroben Schwelle eines Probanden.

Ferner ist die Erfindung auf ein Verfahren zum Überwachen einer blutbezogenen, individuellen anaeroben Schwelle eines Probanden gerichtet.

Außerdem betrifft die Erfindung ein Verfahren zum Trainieren eines Datenmodells zum Bestimmen einer blutbezogenen, individuellen anaeroben Schwelle.

Zudem ist die Erfindung auf eine Vorrichtung zur Datenverarbeitung, ein Computerprogramm und ein computerlesbares Medium gerichtet.

Auch ist die Erfindung auf ein System zum Bestimmen einer blutbezogenen, individuellen anaeroben Schwelle eines Probanden und auf ein System zum Überwachen einer blutbezogenen, individuellen anaeroben Schwelle eines Probanden gerichtet.

In diesem Zusammenhang ist die blutbezogene, individuelle anaerobe Schwelle eines Probanden ein Begriff aus der Sportphysiologie, der einen Energiestoffwechsel des Probanden beschreibt. Die blutbezogene, anaerobe Schwelle, die auch als Schwellenwert bezeichnet werden kann, kann für jeden Probanden unterschiedlich sein und ist somit individuell. Dabei wird die blutbezogene, individuelle anaerobe Schwelle basierend auf einer Laktatkonzentration im Blut des Probanden definiert, weshalb diese individuelle anaerobe Schwelle auch als blutbezogen bezeichnet wird. In diesem Zusammenhang bildet sich das Laktat infolge einer physischen Belastung des Probanden in dessen Muskeln und gelangt von dort aus in dessen Blut. Das bedeutet, dass sich Laktat bildet, wenn der Proband eine Leistung erbringt, wobei sich generell bei höheren Leistungen mehr Laktat bildet als bei niedrigeren Leistungen. Neben der Laktatbildung ist der menschliche Körper auch fähig, Laktat abzubauen. Die anaerobe Schwelle (englisch: anaerobic threshold) beschreibt dabei die höchstmögliche Belastungsintensität oder Leistungsabgabe, welche von einem Probanden gerade noch unter Aufrechterhaltung eines Gleichgewichtszustandes zwischen der Bildung und dem Abbau von Laktat erbracht werden kann. An der anaeroben Schwelle sind also die Bildung und der Abbau von Laktat gerade noch in einem sogenannten Fließgleichgewicht. Das bedeutet für den Probanden, dass eine Leistung unterhalb der anaeroben Schwelle vergleichsweise lange ohne Ermüdung aufrechterhalten werden kann. Oberhalb der anaeroben Schwelle geht die Bildung von Laktat, d. h. die Akkumulation von Laktat im Blut, schneller vonstatten als das Laktat vom menschlichen Körper abgebaut werden kann. Die anaerobe Schwelle wird in der Regel als Leistung oder Leistungswert angegeben. Die Laktatkonzentration wird beispielsweise in mmol/Liter angegeben.

Die blutbezogene, individuelle anaerobe Schwelle ist einerseits bei der Leistungsdiagnostik, d. h. bei der Bewertung der körperlichen Leistungsfähigkeit des Probanden von großer Bedeutung. Andererseits wird die blutbezogene, individuelle anaerobe Schwelle vielfach zur Trainingssteuerung verwendet. Trainingsziel kann dabei sein, die blutbezogene, individuelle anaerobe Schwelle in Richtung zu höheren Werten zu verschieben. Hierfür kann die Trainingsintensität gezielt in Bezug auf die blutbezogene, individuelle anaerobe Schwelle, beispielsweise in Prozent der blutbezogenen, individuellen anaeroben Schwelle, eingestellt werden. Der Proband kann somit sein Training individualisiert und effizient steuern.

Die blutbezogene, individuelle anaerobe Schwelle wird üblicherweise anhand eines sogenannten Stufentests oder Leistungsstufentests ermittelt. Dabei geht der Proband einer sportlichen Aktivität nach, die er zum Beispiel auf einem Ergometer ausführt. Die Leistung wird dabei stufenweise und in regelmäßigen Zeitabständen gesteigert. Beispielsweise startet der Proband bei einer Leistungsabgabe von 70 Watt, die alle drei Minuten um 30 Watt gesteigert wird. Für jede Leistungsstufe wird dem Probanden von einer Hilfsperson eine Blutprobe, z. B. aus dem Finger oder aus dem Ohrläppchen, entnommen, in der die Laktatkonzentration bestimmt wird. Der Leistungsstufentest endet, wenn der Proband wegen Erschöpfung aufgeben muss. Trägt man nun die für jede Leistungsstufe ermittelten Laktatkonzentrationen über die Leistungsstufen und/oder über die Zeit auf, lässt sich anhand des Verlaufs der Laktatkonzentration die blutbezogene, individuelle anaerobe Schwelle bestimmen.

Es versteht sich, dass der Leistungsstufentest nicht nur aus sportlicher Sicht für den Probanden unangenehm sein kann, sondern auch die notwendigen Blutentnahmen für den Probanden schmerzhaft sein können und Aufwand für die Unterstützungsperson beim Leistungsstufentest verursachen.

Die Aufgabe der vorliegenden Erfindung besteht somit darin, das Bestimmen einer blutbezogenen, individuellen anaeroben Schwelle eines Probanden zu vereinfachen.

Die Aufgabe wird durch ein Verfahren zum Bestimmen einer blutbezogenen, individuellen anaeroben Schwelle eines Probanden gelöst, wobei das Verfahren umfasst:
- Erhalten von ersten Daten, welche eine Leistung des Probanden beschreibende Leistungswerte, einen jeweils zugehörigen Erfassungszeitpunkt beschreibende Zeitwerte und jeweils zugehörige Speichellaktatkonzentrationswerte umfassen, wobei die ersten Daten über einen maximalen Speichellaktatkonzentrationswert hinausgehen, und
- Erhalten der blutbezogenen, individuellen anaeroben Schwelle basierend auf den ersten Daten und unter Nutzung eines Datenmodells.

In diesem Zusammenhang meint das Erhalten von ersten Daten ein Ermitteln oder Empfangen der ersten Daten. Im Unterschied zum Stand der Technik verwendet das erfindungsgemäße Verfahren also Speichellaktatkonzentrationswerte, d.h. Werte, welche eine Laktatkonzentration im Speichel des Probanden beschreiben. Eine Entnahme von Blut und eine Blutanalyse können somit entfallen. Das bedeutet sowohl eine Erleichterung für den Probanden als auch eine Erleichterung für die Unterstützungsperson. Die ersten Daten, die die Speichellaktatkonzentrationswerte umfassen, werden unter Nutzung eines Datenmodells in die blutbezogene, individuelle anaerobe Schwelle umgerechnet. Dabei liegt die Erkenntnis zugrunde, dass sich bei einer körperlichen Belastung des Probanden, Laktat auch im Speichel nachweisen lässt. Das bedeutet, dass das aufgrund der Belastung gebildete Laktat auch im Speichel vorliegt und aufgrund physiologischer Abbauprozesse abgebautes Laktat auch zu einem Rückgang der Laktatkonzentration im Speichel führt. Dabei ist eine Speichellaktatkonzentration unter ansonsten gleichen Bedingungen allerdings stets kleiner als eine Blutlaktatkonzentration. Zudem sind die Speichellaktatkonzentration und die Blutlaktatkonzentration zeitversetzt, üblicherweise um 6 bis 10 Minuten. Das im erfindungsgemäßen Verfahren verwendete Datenmodell bildet dabei den Zusammenhang zwischen der Speichellaktatkonzentration und der Blutlaktatkonzentration ab. Die Tatsache, dass die ersten Daten über einen maximalen Speichellaktatkonzentrationswert hinausgehen, bedeutet, dass ein Maximum der Speichellaktatkonzentration in den ersten Daten enthalten ist. Dasselbe gilt für den zur maximalen Speichellaktatkonzentration gehörenden Leistungswert und einen zugehörigen Zeitwert. Dadurch wird berücksichtigt, dass, wie bereits erwähnt, die Speichellaktatkonzentration zeitlich der Blutlaktatkonzentration nachläuft. Für den Fall, dass das erfindungsgemäße Verfahren im Zusammenhang mit einem Leistungsstufentest verwendet wird, bedeutet dies, dass auch nach dem Erreichen der Maximalleistung des Probanden, d. h. nach dem eigentlichen Testabbruch weiterhin die Speichellaktatkonzentration erfasst werden muss. Das ermöglicht es, mittels des bereits erwähnten Datenmodells den Zusammenhang zwischen Speichellaktatkonzentration und Blutlaktatkonzentration vollständig abzubilden. Insgesamt kann also mittels des erfindungsgemäßen Verfahrens eine blutbezogene, individuelle anaerobe Schwelle eines Probanden präzise und zuverlässig ermittelt werden, wobei gleichzeitig auf Blutentnahmen verzichtet werden kann. Auf diese Weise wird das Bestimmen der blutbezogenen, individuellen anaeroben Schwelle einfacher und angenehmer.

Im Zusammenhang mit dem erfindungsgemäßen Verfahren zum Bestimmen der blutbezogenen, individuellen anaeroben Schwelle lassen sich zum Beispiel Speichellaktatkonzentrationswerte mithilfe eines Intraoralsensors, der zur Detektion von Speichellaktatkonzentrationswerten oder einer Speichellaktatkonzentration ausgebildet ist, erfassen. Gleichfalls ist es möglich, im Zusammenhang mit dem erfindungsgemäßen Verfahren zum Bestimmen der blutbezogenen, individuellen anaeroben Schwelle einen extraoralen Sensor zu verwenden. In diesem Fall muss der Proband zur Messung der Speichellaktatkonzentration, Speichel in ein Behältnis überführen, zum Beispiel indem der Proband in das Behältnis spuckt. Es wird dann die Speichellaktatkonzentration im Behältnis gemessen.

Das erfindungsgemäße Verfahren zum Bestimmen der blutbezogenen, individuellen anaeroben Schwelle lässt sich lokal auf einer Datenverarbeitungseinrichtung ausführen, die im Umfeld des Probanden angeordnet ist. Alternativ kann das Verfahren zum Bestimmen der blutbezogenen, individuellen, anaeroben Schwelle auch auf einer vom Probanden weiter entfernten Datenverarbeitungseinrichtung ausgeführt werden, zum Beispiel auf einem Server, insbesondere Cloud-Server. Ebenso sind Mischformen möglich, d. h. dass Teile der Verfahrensschritte des erfindungsgemäßen Verfahrens zum Bestimmen der blutbezogenen, individuellen anaeroben Schwelle lokal, d. h. im Umfeld des Probanden, ausgeführt werden können und andere Teile der Verfahrensschritte auf einem Server ausgeführt werden können.

Das Datenmodell kann ein trainiertes Datenmodell umfassen, welches die ersten Daten als Eingangsdaten verwendet und dazu ausgebildet ist, die blutbezogene, individuelle anaerobe Schwelle auszugeben. Trainingsdaten für ein solches trainiertes Datenmodell umfassen somit Trainingsdatenelemente, die eine blutbezogene Laktatkonzentration und eine speichelbezogene Laktatkonzentration, jeweils mit zugehörigen Leistungswerten und Zeitwerten, umfassen. Beispielsweise umfasst das trainierte Datenmodell ein trainiertes künstliches neuronales Netz.

Ein derartiges Datenmodell ist besonders gut dafür geeignet, Zusammenhänge zwischen der Blutlaktatkonzentration und der Speichellaktatkonzentration zumindest implizit abzubilden. Ferner lässt sich ein derartiges trainiertes Datenmodell in der Ausführung des Verfahrens zum Bestimmen der blutbezogenen, individuellen anaerobe Schwelle einfach und zuverlässig ausführen. Die blutbezogene, individuelle anaerobe Schwelle kann also schnell und präzise ermittelt werden.

In einem Beispiel ist das trainierte Datenmodell spezifisch für einen Leistungsstufentest, eine Sportart und/oder ein Fitnesslevel. Das bedeutet, dass das Datenmodell speziell für den relevanten Leistungsstufentest, die relevante Sportart und/oder das relevante Fitnesslevel trainiert wurde. In diesem Zusammenhang können sich Leistungsstufentests beispielsweise durch die Größe der verwendeten Zeit- und Belastungsinkremente unterscheiden. Dadurch kann das trainierte Datenmodell für diesen Leistungsstufentest, diese Sportart und/oder dieses Fitnesslevel besonders präzise sein. In diesem Zusammenhang ist es folglich auch möglich, mehrere trainierte Datenmodelle vorzusehen, wobei jedes der Datenmodelle spezifisch für einen Leistungsstufentest, eine Sportart und/oder ein Fitnesslevel ist. Somit kann bei der Ausführung des Verfahrens zum Bestimmen der blutbezogenen, individuellen anaeroben Schwelle stets das passende trainierte Datenmodell ausgewählt und angewendet werden. Es wird also stets dasjenige Datenmodell verwendet, das für den relevanten Leistungsstufentest, die relevante Sportart und/oder das relevante Fitnesslevel trainiert wurde. Beispielsweise können unterschiedliche Datenmodelle für die Sportarten Schwimmen, Laufen, Radfahren und Rudern vorgesehen sein. In gleicher Weise können verschiedene Datenmodelle für die Fitnesslevels unsportlich, Hobbysportler und Profisportler vorgesehen sein. Insgesamt kann also erreicht werden, dass das Verfahren zum Bestimmen der blutbezogenen, individuellen anaeroben Schwelle in einer Vielzahl an Anwendungsfällen stets präzise und zuverlässig arbeitet. Optional kann das trainierte Datenmodell zudem Informationen zum Alter des Probanden, der Größe des Probanden und/oder zum Gewicht des Probanden nutzen. Allgemeiner gesprochen kann das trainierte Datenmodell Anamnesedaten nutzen, die den Probanden beschreiben. In diesem Zusammenhang können die Anamnesedaten als zusätzliche Parameter eines trainierten Datenmodells verwendet werden. Das setzt selbstverständlich voraus, dass auch beim Training des Datenmodells Anamnesedaten berücksichtigt werden. Alternativ können eines oder mehrere trainierte Datenmodelle vorgesehen werden, die spezifisch für eine Altersklasse, einen Größenbereich und/oder einen Gewichtsbereich von Probanden sind. Allgemeiner gesprochen, können trainierte Datenmodelle spezifisch für gewisse Probandengruppen sein, wobei die Probandengruppen basierend auf Anamnesedaten definiert werden. Durch Nutzung dieser Optionen kann das Verfahren zum Bestimmen der blutbezogenen, individuellen anaeroben Schwelle in einer Vielzahl an Anwendungsfällen besonders präzise und zuverlässig arbeiten.

Auch kann das Datenmodell ein Regressionsmodell umfassen, welches einen Zusammenhang zwischen der blutbezogenen, individuellen anaeroben Schwelle und einer speichelbezogenen, individuellen anaeroben Schwelle beschreibt. Folglich kann die speichelbezogene, individuelle anaerobe Schwelle einfach und zuverlässig in eine blutbezogene, individuelle anaerobe Schwelle umgerechnet werden. Dabei kann die speichelbezogene, individuelle anaerobe Schwelle basierend auf den ersten Daten ermittelt werden. Auf diese Weise lässt sich die blutbezogene, individuelle anaerobe Schwelle präzise und zuverlässig bestimmen, ohne dass hierfür eine Entnahme oder Analyse von Blut notwendig ist.

Es versteht sich, dass es zum Erstellen des Regressionsmodells, welches einen Zusammenhang zwischen der blutbezogenen, individuellen anaeroben Schwelle und einer speichelbezogenen, individuellen anaeroben Schwelle beschreibt, notwendig sein kann, bei einer Mehrzahl an Probanden sowohl die blutbezogene, individuelle anaerobe Schwelle als auch die speichelbezogene, individuelle anaerobe Schwelle zu bestimmen. Hierfür kann beispielsweise ein Leistungsstufentest der oben beschriebenen Art verwendet werden. Die Bestimmung der blutbezogenen, individuellen anaeroben Schwelle kann auf tatsächlichen Blutproben basieren. Folglich kann basierend auf der Mehrzahl an Probanden der Zusammenhang zwischen der blutbezogenen, individuellen anaeroben Schwelle und der speichelbezogenen, individuellen anaeroben Schwelle bestimmt werden. Dieser Zusammenhang kann dann in Form des Regressionsmodells für das Bestimmen der blutbezogenen, individuellen anaeroben Schwelle verwendet werden, wofür dann keine Entnahme oder Untersuchung von Blutproben mehr nötig ist.

Gemäß einer Variante ist es möglich, mehrere Regressionsmodelle, welche einen Zusammenhang zwischen einer blutbezogenen, individuellen anaeroben Schwelle und einer speichelbezogenen, individuellen anaeroben Schwelle beschreiben, als Alternativen bereitzustellen und im Zuge des Verfahrens zum Bestimmen der blutbezogenen, individuellen anaeroben Schwelle das jeweils passende Regressionsmodell auszuwählen. Wie bereits oben im Zusammenhang mit dem trainierten Datenmodell beschrieben, können die unterschiedlichen Regressionsmodelle unterschiedliche Leistungsstufentests, unterschiedliche Sportarten und/oder unterschiedliche Fitnesslevels betreffen.

Alternativ oder zusätzlich umfasst das Datenmodell sowohl ein Regressionsmodell, das einen Zusammenhang zwischen der blutbezogenen, individuellen aeroben Schwelle und einer speichelbezogenen, individuellen aeroben Schwelle beschreibt, als auch ein Regressionsmodell, das einen Zusammenhang zwischen einem maximalen Speichellaktatkonzentrationswert und einem maximalen Blutlaktatkonzentrationswert beschreibt. In diesem Zusammenhang beschreibt die aerobe Schwelle (englisch: aerobic threshold) eine Leistung des Probanden, bei der die Laktatkonzentration im Blut zwar über eine Ruhe-Laktatkonzentration ansteigt, aber noch durch die körpereigenen Abbauprozesse im bereits erwähnten Fließgleichgewicht gehalten werden kann. Mit anderen Worten beschreibt die aerobe Schwelle eine niedrigste Belastungsintensität oder niedrigste abgegebene Leistung des Probanden, bei der die Muskulatur nicht mehr rein aerob arbeitet. Mittels eines Regressionsmodells, das einen Zusammenhang zwischen der blutbezogenen, individuellen aeroben Schwelle und einer speichelbezogenen, individuellen aeroben Schwelle beschreibt, kann die speichelbezogene, individuelle aerobe Schwelle einfach und zuverlässig in eine blutbezogene, individuelle aerobe Schwelle umgerechnet werden. Dabei kann die speichelbezogene, individuelle aerobe Schwelle basierend auf den ersten Daten ermittelt werden. Mittels eines Regressionsmodells, das einen Zusammenhang zwischen einem maximalen Speichellaktatkonzentrationswert und einem maximalen Blutlaktatkonzentrationswert beschreibt, kann ferner die maximale Speichellaktatkonzentration einfach und zuverlässig in eine maximale Blutlaktatkonzentration umgerechnet werden. Dabei kann die maximale Speichellaktatkonzentration basierend auf den ersten Daten ermittelt werden. Auf diese Weise lassen sich also die blutbezogene, individuelle aerobe Schwelle und die maximale Blutlaktatkonzentration präzise und zuverlässig bestimmen, ohne dass hierfür eine Entnahme oder Analyse von Blut notwendig ist. Sodann kann basierend auf dem maximalen Blutkonzentrationswert und der blutbezogenen, individuellen aeroben Schwelle, beispielsweise unter Nutzung eines sogenannten D-max-Verfahrens die blutbezogene anaerobe Schwelle ermittelt werden. Hierzu kann es zudem nötig sein, Speichellaktatkonzentrationswerte, die zwischen der speichelbezogenen, individuellen aeroben Schwelle und dem maximalen Speichellaktatkonzentrationswert liegen, in Blutlaktatkonzentrationswerte umzurechnen. Dabei können die Speichellaktatkonzentrationswerte einzeln in Blutlaktatkonzentrationswerte umgerechnet werden. Optional kann dann eine Kurve durch die Blutlaktatkonzentrationswerte gelegt werden, z. B. im Zuge eines Fittings. Alternativ kann eine Kurve durch die Speichellaktatkonzentrationswerte gelegt werden, z. B. im Zuge eines Fittings, und sodann die Kurve, die die Speichellaktatkonzentrationswerte beschreibt, in eine Kurve umgerechnet werden, die Blutlaktatkonzentrationswerte beschreibt. Die Umrechnung von Speichellaktatkonzentrationswerten oder einer Speichellaktatkonzentrationswerte beschreibenden Kurve in Blutlaktatkonzentrationswerte oder eine Blutlaktatkonzentrationswerte beschreibende Kurve kann zum Beispiel durch ein erstes Offset entlang einer Zeitachse und durch ein zweites Offset entlang einer Laktatkonzentrationsachse erfolgen. Dabei wird durch das erste Offset der bereits erwähnte zeitliche Versatz zwischen den Blutlaktatkonzentrationswerten und den Speichellaktatkonzentrationswerten berücksichtigt. Das zweite Offset berücksichtigt, dass, wie bereits erwähnt, Speichellaktatkonzentrationswerte generell niedriger sind als Blutlaktatkonzentrationswerte. Alternativ hierzu kann die Umrechnung von Speichellaktatkonzentrationswerten oder einer Speichellaktatkonzentrationswerte beschreibenden Kurve in Blutlaktatkonzentrationswerte oder eine Blutlaktatkonzentrationswerte beschreibende Kurve anhand eines weiteren Datenmodells, insbesondere eines weiteren Regressionsmodells, erfolgen. Im Detail wird beim Ausführen des D-max-Verfahrens zunächst eine Gerade erstellt, die sich zwischen der maximalen Blutlaktatkonzentration und derjenigen Blutlaktatkonzentration, die an der blutbezogenen, aeroben individuellen Schwelle auftritt, erstreckt. Ausgehend von dieser Geraden wird derjenige der Blutlaktatkonzentrationswerte ermittelt, der den größten Abstand von dieser Geraden hat, wobei der Abstand rechtwinklig zur Geraden gemessen wird. Die zu dieser Blutlaktatkonzentration gehörende Leistung entspricht der blutbezogenen anaeroben Schwelle. Es versteht sich, dass im Zuge des D-max-Verfahrens anstelle des Blutlaktatkonzentrationswerts, der den größten Abstand von der Geraden hat, auch der Blutlaktatkonzentrationswert gesucht werden kann, der die gleiche Steigung wie die Gerade aufweist. Das Ergebnis ist dasselbe. Es lässt sich also auch auf diese Weise die blutbezogene, individuelle anaerobe Schwelle ermitteln, ohne dass dafür Blutproben entnommen oder analysiert werden müssen.

Es versteht sich, dass es zum Erstellen des Regressionsmodells, welches einen Zusammenhang zwischen der blutbezogenen, individuellen aeroben Schwelle und einer speichelbezogenen, individuellen aeroben Schwelle beschreibt, notwendig sein kann, bei einer Mehrzahl an Probanden sowohl die blutbezogene, individuelle aerobe Schwelle als auch die speichelbezogene, individuelle aerobe Schwelle zu bestimmen. Hierfür kann beispielsweise ein Leistungsstufentest der oben beschriebenen Art verwendet werden. Die Bestimmung der blutbezogenen, individuellen aeroben Schwelle kann auf tatsächlichen Blutproben basieren. Folglich kann basierend auf der Mehrzahl an Probanden der Zusammenhang zwischen der blutbezogenen, individuellen aeroben Schwelle und der speichelbezogenen, individuellen aeroben Schwelle bestimmt werden. Dieser Zusammenhang kann dann in Form des Regressionsmodells für das Bestimmen der blutbezogenen, individuellen aeroben Schwelle verwendet werden, wofür dann keine Entnahme oder Untersuchung von Blutproben mehr nötig ist.

Gemäß einer Variante ist es auch hier möglich, mehrere Regressionsmodelle, welche einen Zusammenhang zwischen der blutbezogenen, individuellen aeroben Schwelle und einer speichelbezogenen, individuellen aeroben Schwelle beschreiben, als Alternativen bereitzustellen und im Zuge des Verfahrens zum Bestimmen der blutbezogenen, individuellen anaeroben Schwelle das jeweils passende Regressionsmodell auszuwählen. Wie bereits oben im Zusammenhang mit dem trainierten Datenmodell beschrieben, können die unterschiedlichen Regressionsmodelle unterschiedliche Leistungsstufentests, unterschiedliche Sportarten und/oder unterschiedliche Fitnesslevels betreffen.

Es versteht sich ferner, dass es zum Erstellen des Regressionsmodells, welches einen Zusammenhang zwischen dem maximalen Blutlaktatkonzentrationswert und dem maximalen Speichellaktatkonzentrationswert beschreibt, notwendig sein kann, bei einer Mehrzahl an Probanden sowohl den maximalen Blutlaktatkonzentrationswert als auch den maximalen Speichellaktatkonzentrationswert zu bestimmen. Hierfür kann beispielsweise ein Leistungsstufentest der oben beschriebenen Art verwendet werden. Die Bestimmung des maximalen Blutlaktatkonzentrationswerts kann auf tatsächlichen Blutproben basieren. Folglich kann basierend auf der Mehrzahl an Probanden der Zusammenhang zwischen dem maximalen Blutlaktatkonzentrationswert und dem maximalen Speichellaktatkonzentrationswert bestimmt werden. Dieser Zusammenhang kann dann in Form des Regressionsmodells für das Bestimmen des maximalen Blutlaktatkonzentrationswerts verwendet werden, wofür dann keine Entnahme oder Untersuchung von Blutproben mehr nötig ist.

Gemäß einer Variante ist es auch hier möglich, mehrere Regressionsmodelle, welche einen Zusammenhang zwischen dem maximalen Blutlaktatkonzentrationswert und dem maximalen Speichellaktatkonzentrationswert beschreiben, als Alternativen bereitzustellen und im Zuge des Verfahrens zum Bestimmen der blutbezogenen, individuellen anaeroben Schwelle das jeweils passende Regressionsmodell auszuwählen. Wie bereits oben im Zusammenhang mit dem trainierten Datenmodell beschrieben, können die unterschiedlichen Regressionsmodelle unterschiedliche Leistungsstufentests, unterschiedliche Sportarten und/oder unterschiedliche Fitnesslevels betreffen.

Bei allen zuvor erläuterten Regressionsmodellen kann es sich um lineare Regressionsmodelle oder um Polynom-Regressionsmodelle oder um Exponentialregressionsmodelle handeln. Optional kann beim Erstellen der erläuterten Regressionsmodelle die sogenannte Pearson-Korrelationsanalyse verwendet werden. Mittels derartiger Regressionsmodelle lässt sich ein Zusammenhang zwischen der blutbezogenen, individuellen anaeroben Schwelle und der speichelbezogenen, individuellen anaeroben Schwelle und/oder ein Zusammenhang zwischen der blutbezogenen, individuellen aeroben Schwelle und einer speichelbezogenen, individuellen aeroben Schwelle und/oder ein Zusammenhang zwischen einem maximalen Speichellaktatkonzentrationswert und einem maximalen Blutlaktatkonzentrationswert einfach und zuverlässig beschreiben.

Gemäß einer Ausführungsform umfasst das Erhalten der blutbezogenen, individuellen anaeroben Schwelle ein Erhalten einer speichelbezogenen, individuellen anaeroben Schwelle basierend auf den ersten Daten. Das bedeutet, dass basierend auf den ersten Daten eine speichelbezogene, individuelle anaerobe Schwelle errechnet wird. Diese speichelbezogene, individuelle anaerobe Schwelle wird dann verwendet, um die blutbezogene, individuelle anaerobe Schwelle zu erhalten. Es muss also sozusagen die Laktatkonzentration nur noch vom Speichel ins Blut umgerechnet werden. Wie bereits erwähnt, kann hierfür ein Datenmodell, zum Beispiel ein Regressionsmodell, verwendet werden. Insgesamt lässt sich einfach und zuverlässig die blutbezogene, individuelle anaerobe Schwelle erhalten.

Dabei kann das Erhalten der speichelbezogenen, individuellen anaeroben Schwelle ein Erhalten einer speichelbezogenen, individuellen aeroben Schwelle umfassen. Die speichelbezogene anaerobe Schwelle wird also basierend auf der speichelbezogenen aeroben Schwelle ermittelt.

Die speichelbezogene, individuelle aerobe Schwelle kann als derjenige Leistungswert erhalten werden, bei dem ein Quotient aus Speichellaktatkonzentrationswert und zugehörigem Leistungswert minimal ist. Alternativ kann die speichelbezogene, individuelle aerobe Schwelle ein Auswerten einer zeitlichen Änderungsrate der Speichellaktatkonzentrationswerte umfassen. In der ersten Alternative wird also virtuell oder tatsächlich ein Quotient aus Speichellaktatkonzentrationswert und Leistungswert über eine Leistung aufgetragen. Diejenige Leistung, d. h. derjenige Leistungswert, bei dem der Quotient minimal ist, stellt die speichelbezogene, individuelle aerobe Schwelle dar. Auf diese Weise lässt sich die speichelbezogene, individuelle aerobe Schwelle einfach und zuverlässig berechnen. Gemäß der zweiten Alternative wird eine Änderungsrate der Speichellaktatkonzentrationswerte ausgewertet. Wie bereits erwähnt, ist die aerobe Schwelle durch denjenigen kleinsten Leistungswert gebildet, bei dem eine Laktatkonzentration über eine Ruhe-Laktatkonzentration ansteigt. Mit anderen Worten kann die speichelbezogene, aerobe Schwelle als diejenige Leistung ermittelt werden, bei der der Gradient der Laktatkonzentration erstmals wesentlich von Null abweicht. Dabei kann eine wesentliche Abweichung dadurch definiert sein, dass der Gradient um mindestens einen vorgegebenen Wert von Null abweicht. Alternativ ist es möglich, einen Grenzwert oder ein Intervall für den Gradienten zu bestimmen, bei dessen Erreichen die speichelbezogene, individuelle aerobe Schwelle definiert wird. Auch im Rahmen dieser Alternative lässt sich die speichelbezogene, individuelle aerobe Schwelle einfach und zuverlässig ermitteln.

Für den Fall, dass in der oben beschriebenen Variante Grenzwerte oder Intervalle für den Gradienten vordefiniert sind, können diese Grenzwerte oder Intervalle bestimmt werden, indem beispielsweise während eines Leistungsstufentest, für eine Mehrzahl an Probanden über ein gewisses Leistungsspektrum Laktatkonzentrationen im Speichel ermittelt werden und gleichzeitig mittels an sich bekannter Verfahren für jeden Probanden die speichelbezogene, individuelle aerobe Schwelle ermittelt wird. Basierend hierauf kann dann für jeden Probanden ermittelt werden, welchen Gradienten die Laktatkonzentration an der speichelbezogenen, aeroben Schwelle aufweist. Die Gradienten, die anhand verschiedener Probanden ermittelt werden, können dann aggregiert werden, zum Beispiel indem ein Mittelwert gebildet wird. Derartige aggregierte Werte, können dann als Grenzwerte dienen und/oder zur Definition von Intervallen herangezogen werden.

In einem Beispiel umfasst das Erhalten der speichelbezogenen, individuellen anaeroben Schwelle ein Ermitteln eines maximalen Abstands einer Geraden von einer die Speichellaktatkonzentrationswerte verbindenden Kurve. Dabei erstreckt sich die Gerade von einem minimalen Speichellaktatkonzentrationswert zum maximalen Speichellaktatkonzentrationswert oder von einem der individuellen aeroben Schwelle zugeordneten Speichellaktatkonzentrationswert zum maximalen Speichellaktatkonzentrationswert. Die speichelbezogene, individuelle anaerobe Schwelle wird als Leistungswert am Punkt des maximalen Abstands erhalten. Es versteht sich, dass im Zuge des D-max-Verfahrens anstelle des Speichellaktatkonzentrationswerts, der den größten Abstand von der Geraden hat, auch der Speichellaktatkonzentrationswert gesucht werden kann, der die gleiche Steigung wie die Gerade aufweist. Das Ergebnis ist dasselbe. In diesem Beispiel wird also ein sogenanntes D-max-Verfahren auf die Speichellaktatkonzentrationswerte angewendet. Auch auf diese Weise lässt sich die speichelbezogene, individuelle anaerobe Schwelle einfach und zuverlässig ermitteln.

In einer Variante kann das Erhalten der speichelbezogenen, individuellen anaeroben Schwelle ein Auswerten einer zeitlichen Änderungsrate der Speichellaktatkonzentrationswerte umfassen. Wie bereits erläutert, ist die anaerobe Schwelle dadurch definiert, dass Laktat derart schnell generiert wird, dass es durch die körpereigenen Abbauprozesse nicht mehr im Fließgleichgewicht gehalten werden kann. Das bedeutet, dass an der anaeroben Schwelle ein Gradient der Speichellaktatkonzentration ausgehend von einem im Wesentlichen konstanten Wert beginnt zu steigen. Das lässt sich entweder anhand einer virtuellen oder tatsächlichen Kurve, die Laktatkonzentrationen beschreibt, ermitteln. Alternativ kann ein vordefinierter Grenzwert für den Gradient verwendet werden, bei dessen Erreichen vom Erreichen der anaeroben Schwelle ausgegangen wird. Ein derartiger Grenzwert kann bestimmt werden, indem beispielsweise während eines Leistungsstufentests, für eine Mehrzahl an Probanden über ein gewisses Leistungsspektrum Laktatkonzentrationen im Speichel ermittelt werden und gleichzeitig mittels an sich bekannter Verfahren für jeden Probanden die speichelbezogene, individuelle anaerobe Schwelle ermittelt wird. Basierend hierauf kann dann für jeden Probanden ermittelt werden, welchen Gradienten die Laktatkonzentration an der speichelbezogenen, anaeroben Schwelle aufweist. Die Gradienten, die anhand verschiedener Probanden ermittelt werden, können dann aggregiert werden, zum Beispiel indem ein Mittelwert gebildet wird. Derartige aggregierte Werte, können dann als Grenzwerte dienen.

Zusätzlich wird die Aufgabe durch ein Verfahren zum Überwachen einer blutbezogenen, individuellen anaeroben Schwelle eines Probanden gelöst. Das Verfahren umfasst:
- Erhalten von zweiten Daten, welche Speichellaktatkonzentrationswerte und einen jeweils zugehörigen Erfassungszeitpunkt beschreibende Zeitwerte umfassen,
- Erhalten der blutbezogenen, individuellen anaeroben Schwelle des Probanden, wobei die blutbezogene, individuelle anaerobe Schwelle des Probanden mittels des erfindungsgemäßen Verfahrens zum Bestimmen einer blutbezogenen, individuellen anaeroben Schwelle eines Probanden ermittelt ist, oder Erhalten einer zeitlichen Änderungsrate der Speichellaktatkonzentrationswerte und Erhalten einer speichelbezogenen, individuellen anaeroben Schwelle basierend auf der zeitlichen Änderungsrate der Speichellaktatkonzentrationswerte, und
- Bereitstellen eines Indikators, welcher eine Beziehung der zweiten Daten zur speichelbezogenen, individuellen anaeroben Schwelle oder zur blutbezogenen, individuellen anaeroben Schwelle beschreibt.

Der Indikator kann in diesem Zusammenhang beispielsweise darauf hinweisen, dass die speichelbezogene, individuelle anaerobe Schwelle oder die blutbezogene, individuelle anaerobe Schwelle unterschritten, überschritten oder erreicht ist. Der Indikator gibt also für einen Probanden an, wie sich seine Leistung zur speichelbezogenen, individuellen anaeroben Schwelle oder zur blutbezogenen, individuellen anaeroben Schwelle verhält. Anders gesagt gibt der Indikator für den Probanden an, über welche dominierenden Stoffwechselmechanismen er die Leistung bereitstellt. Auf diese Weise wird dem Probanden ermöglicht, seine Belastungen, d. h. die abgegebene Leistung, beispielsweise zu Trainingszwecken, präzise einzustellen. Alternativ oder zusätzlich kann der Proband auf diese Weise seine Belastungen, d.h. beispielsweise sein Training, dahingehend dokumentieren, dass festgehalten wird, über welche Zeiträume oder Zeitanteile die speichelbezogene, individuelle anaerobe Schwelle oder die blutbezogene, individuelle anaerobe Schwelle unterschritten, überschritten oder erreicht war.

Wie bereits erwähnt, läuft die Speichellaktatkonzentration der Blutlaktatkonzentration zeitlich üblicherweise um 6 bis 10 Minuten nach. Es hat sich jedoch herausgestellt, dass es trotz dieses Zeitversatzes sinnvoll sein kann, eine von einem Probanden abgegebene Leistung, beispielsweise zu Trainingszwecken, rein basierend auf zweiten Daten, welche Speichellaktatkonzentrationswerte und einen jeweils zugehörigen Erfassungszeitpunkt beschreibende Zeitwerte umfassen, sowie auf einer auf dieser Basis erhaltenen speichelbezogenen, individuellen anaeroben Schwelle zu steuern. Bezüglich der Blutlaktatkonzentration kann ein Unterschreiten, Überschreiten oder Erreichen der speichelbezogenen, individuellen anaeroben Schwelle zwar nur mit einem gewissen Zeitversatz mittels des Indikators angezeigt werden. Dennoch ist diese Information für die Belastungssteuerung und die Belastungsdokumentation wertvoll. Dies gilt umso mehr, je weniger die Belastung über den Belastungszeitraum schwankt und je länger der Belastungszeitraum ist. Vereinfacht gesagt, wird der Einfluss des Zeitversatzes, mit dem die speichelbezogene, individuelle anaerobe Schwelle ermittelt werden kann, geringer, je länger der Proband eine im Wesentlichen konstante Belastung erfährt, d.h. eine im Wesentlichen konstante Belastung abgibt. Bei der Belastungsdokumentation ist der Zeitversatz irrelevant, da nach einer Wartezeit von üblicherweise 6 bis 10 Minuten stets eine vollständige Belastungsdokumentation vorliegt.

Gemäß einer Variante des Verfahrens zum Überwachen einer blutbezogenen, individuellen anaeroben Schwelle eines Probanden kann das Erhalten einer zeitlichen Änderungsrate der Speichellaktatkonzentrationswerte und Erhalten einer speichelbezogenen, individuellen anaeroben Schwelle basierend auf der zeitlichen Änderungsrate der Speichellaktatkonzentrationswerte optional ein Vorhersagen oder Schätzen der blutbezogenen, individuellen anaeroben Schwelle umfassen. Mit anderen Worten kann die blutbezogene, individuelle anaerobe Schwelle basierend auf den zweiten Daten, der zeitlichen Änderungsrate der Speichellaktatkonzentrationswerte und/oder der speichelbezogenen, individuellen anaeroben Schwelle vorhergesagt oder abgeschätzt werden. Hierfür kann wieder ein Datenmodell verwendet werden. Dieses Datenmodell kann ein Regressionsmodell umfassen. Dabei kann es zum Erstellen des Regressionsmodells notwendig sein, bei einer Mehrzahl an Probanden sowohl die blutbezogene, individuelle anaerobe Schwelle als auch die speichelbezogene, individuelle anaerobe Schwelle zu bestimmen, wie bereits beschrieben wurde. Alternativ kann das Regressionsmodell basierend auf historischen, d.h. in der Vergangenheit liegenden, Belastungen des Probanden, erstellt werden. Alternativ oder zusätzlich zum Regressionsmodell kann auch ein trainiertes Datenmodell verwendet werden. Analog zum Regressionsmodell kann dabei das Training mittels Trainingsdaten erfolgen, die anhand einer Mehrzahl an Probanden ermittelt wurden. Alternativ oder zusätzlich können die Trainingsdaten basierend auf historischen, d.h. in der Vergangenheit liegenden, Belastungen des Probanden, ermittelt werden.

Ferner wird die Aufgabe durch ein Verfahren zum Trainieren eines Datenmodells zum Bestimmen einer blutbezogenen, individuellen anaeroben Schwelle gelöst. Das Datenmodell wird mittels eines annotierten Datensatzes trainiert, welcher mehrere Trainingsdatenelemente umfasst, welche eine Leistung beschreibende Leistungswerte, einen jeweils zugehörigen Erfassungszeitpunkt beschreibende Zeitwerte und jeweils zugehörige Speichellaktatkonzentrationswerte umfassen, wobei die Trainingsdatenelemente mit zugehörigen blutbezogenen, individuellen anaeroben Schwellen annotiert sind. Die Trainingsdatenelemente entsprechen also inhaltlich den zuvor beschriebenen ersten Daten.

Mittels eines derart trainierten Datenmodells lässt sich, wie bereits erläutert, einfach und zuverlässig anhand von ersten Daten, welche eine Leistung des Probanden beschreibende Leistungswerte, einen jeweils zugehörigen Erfassungszeitpunkt beschreibende Zeitwerte und jeweils zugehörige Speichellaktatkonzentrationswerte umfassen, eine blutbezogene, individuelle anaeroben Schwelle erhalten, ohne dass dafür Blutproben genommen und/oder analysiert werden müssen. Mit anderen Worten erlaubt ein derartig trainiertes Datenmodell, lediglich anhand von Messungen im Speichel eine blutbezogene, individuelle anaerobe Schwelle zu erhalten.

Gemäß einer Variante kann das Datenmodell Teilmodelle umfassen, die separat trainiert werden können.

Ein derartiges Teilmodell kann dahingehend trainiert werden, dass es zum Bestimmen einer blutbezogenen, individuellen aeroben Schwelle ausgebildet ist. Es wird also ein Verfahren zum Trainieren eines Datenmodells, genauer gesagt des erwähnten Teilmodells, zum Bestimmen einer blutbezogenen, individuellen aeroben Schwelle ausgeführt. Das Teilmodell wird mittels eines annotierten Datensatzes trainiert, welcher mehrere Trainingsdatenelemente umfasst, welche eine Leistung beschreibende Leistungswerte, einen jeweils zugehörigen Erfassungszeitpunkt beschreibende Zeitwerte und jeweils zugehörige Speichellaktatkonzentrationswerte umfassen, wobei die Trainingsdatenelemente mit zugehörigen blutbezogenen, individuellen aeroben Schwellen annotiert sind. Die Trainingsdatenelemente entsprechen also inhaltlich den zuvor beschriebenen ersten Daten. Mittels eines derart trainierten Datenmodells lässt sich, wie bereits erläutert, einfach und zuverlässig anhand von ersten Daten, welche eine Leistung des Probanden beschreibende Leistungswerte, einen jeweils zugehörigen Erfassungszeitpunkt beschreibende Zeitwerte und jeweils zugehörige Speichellaktatkonzentrationswerte umfassen, eine blutbezogene, individuelle aeroben Schwelle erhalten, ohne dass dafür Blutproben genommen und/oder analysiert werden müssen. Mit anderen Worten erlaubt ein derartig trainiertes Datenmodell, lediglich anhand von Messungen im Speichel eine blutbezogene, individuelle aerobe Schwelle zu erhalten.

Ein anderes Teilmodell kann dahingehend trainiert werden, dass es zum Bestimmen eines maximalen Blutlaktatkonzentrationswerts ausgebildet ist. Es wird also ein Verfahren zum Trainieren eines Datenmodells, genauer gesagt des erwähnten Teilmodells, zum Bestimmen eines maximalen Blutlaktatkonzentrationswerts ausgeführt. Das Teilmodell wird mittels eines annotierten Datensatzes trainiert, welcher mehrere Trainingsdatenelemente umfasst, welche eine Leistung beschreibende Leistungswerte, einen jeweils zugehörigen Erfassungszeitpunkt beschreibende Zeitwerte und jeweils zugehörige Speichellaktatkonzentrationswerte umfassen, wobei die Trainingsdatenelemente mit zugehörigen maximalen Blutlaktatkonzentrationswerten annotiert sind. Die Trainingsdatenelemente entsprechen also inhaltlich den zuvor beschriebenen ersten Daten. Mittels eines derart trainierten Datenmodells lässt sich, wie bereits erläutert, einfach und zuverlässig anhand von ersten Daten, welche eine Leistung des Probanden beschreibende Leistungswerte, einen jeweils zugehörigen Erfassungszeitpunkt beschreibende Zeitwerte und jeweils zugehörige Speichellaktatkonzentrationswerte umfassen, ein maximaler Blutlaktatkonzentrationswert erhalten, ohne dass dafür Blutproben genommen und/oder analysiert werden müssen. Mit anderen Worten erlaubt ein derartig trainiertes Datenmodell, lediglich anhand von Messungen im Speichel einen maximalen Blutlaktatkonzentrationswert zu erhalten.

Ein weiteres Teilmodell kann dahingehend trainiert werden, dass es zum Umrechnen von Speichellaktatkonzentrationswerten in Blutlaktatkonzentrationswerte ausgebildet ist. Es wird also ein Verfahren zum Trainieren eines Datenmodells, genauer gesagt des erwähnten Teilmodells, zum Umrechnen von Speichellaktatkonzentrationswerten in Blutlaktatkonzentrationswerte ausgeführt. Das Teilmodell wird mittels eines annotierten Datensatzes trainiert, welcher mehrere Trainingsdatenelemente umfasst, welche eine Leistung beschreibende Leistungswerte, einen jeweils zugehörigen Erfassungszeitpunkt beschreibende Zeitwerte und jeweils zugehörige Speichellaktatkonzentrationswerte umfassen, wobei die Trainingsdatenelemente mit zugehörigen Blutlaktatkonzentrationswerten annotiert sind. Die Trainingsdatenelemente entsprechen also inhaltlich den zuvor beschriebenen ersten Daten. Mittels eines derart trainierten Datenmodells lassen sich, wie bereits erläutert, einfach und zuverlässig anhand von ersten Daten, welche eine Leistung des Probanden beschreibende Leistungswerte, einen jeweils zugehörigen Erfassungszeitpunkt beschreibende Zeitwerte und jeweils zugehörige Speichellaktatkonzentrationswerte umfassen, zugehörige Blutlaktatkonzentrationswerte erhalten, ohne dass dafür Blutproben genommen und/oder analysiert werden müssen. Mit anderen Worten erlaubt ein derartig trainiertes Datenmodell, lediglich anhand von Messungen im Speichel Blutlaktatkonzentrationswerte zu erhalten.

Es versteht sich, dass insbesondere die trainierten Teilmodelle in Kombination mit dem bereits erläuterten D-max-Verfahren verwendet werden können, um so beispielsweise eine blutbezogene, individuelle anaerobe Schwelle zu ermitteln.

In einem Beispiel ist das Training des Datenmodells, d.h. auch der möglicherweise vorhandenen Teilmodelle, spezifisch für einen Leistungsstufentest, eine Sportart und/oder ein Fitnesslevel. Das bedeutet, dass das Datenmodell speziell für den relevanten Leistungsstufentest, die relevante Sportart und/oder das relevante Fitnesslevel trainiert wird. In diesem Zusammenhang können sich Leistungsstufentests beispielsweise durch die Größe der verwendeten Zeit- und Belastungsinkremente unterscheiden. Dadurch kann das trainierte Datenmodell für diesen Leistungsstufentest, diese Sportart und/oder dieses Fitnesslevel besonders präzise sein. In diesem Zusammenhang ist es folglich auch möglich, mehrere trainierte Datenmodelle vorzusehen, wobei jedes der Datenmodelle spezifisch für einen Leistungsstufentest, eine Sportart und/oder ein Fitnesslevel ist. Somit kann bei der Ausführung des Verfahrens zum Bestimmen der blutbezogenen, individuellen anaeroben Schwelle stets das passende trainierte Datenmodell ausgewählt und angewendet werden. Es wird also stets dasjenige Datenmodell verwendet, das für den relevanten Leistungsstufentest, die relevante Sportart und das relevante Fitnesslevel trainiert wurde. Beispielsweise können unterschiedliche Datenmodelle für die Sportarten Schwimmen, Laufen, Radfahren und Rudern vorgesehen sein.

In gleicher Weise können verschiedene Datenmodelle für die Fitnesslevels unsportlich, Hobbysportler und Profisportler vorgesehen sein. Insgesamt kann also erreicht werden, dass das Verfahren zum Bestimmen der blutbezogenen, individuellen anaeroben Schwelle in einer Vielzahl an Anwendungsfällen stets präzise und zuverlässig arbeitet.

Auch wird die Aufgabe durch eine Vorrichtung zur Datenverarbeitung, umfassend Mittel zur Ausführung wenigstens eines der erfindungsgemäßen Verfahren gelöst. Die Vorrichtung zur Datenverarbeitung umfasst also in einer ersten Alternative Mittel zur Ausführung des erfindungsgemäßen Verfahrens zum Bestimmen einer blutbezogenen, individuellen anaeroben Schwelle eines Probanden. In einer zweiten Alternative umfasst die Vorrichtung zur Datenverarbeitung Mittel zur Ausführung des erfindungsgemäßen Verfahrens zum Überwachen einer blutbezogenen, individuellen anaeroben Schwelle eines Probanden. In einer dritten Alternative umfasst die Vorrichtung zur Datenverarbeitung sowohl Mittel zur Ausführung des erfindungsgemäßen Verfahrens zum Bestimmen einer blutbezogenen, individuellen anaeroben Schwelle eines Probanden, als auch Mittel zur Ausführung des erfindungsgemäßen Verfahrens zum Überwachen einer blutbezogenen, individuellen anaeroben Schwelle eines Probanden. Mittels der erfindungsgemäßen Vorrichtung zur Datenverarbeitung kann also eine blutbezogene, individuelle anaerobe Schwelle eines Probanden präzise und zuverlässig erhalten werden, wobei gleichzeitig auf Blutentnahmen und -analysen verzichtet werden kann. Auf diese Weise wird das Bestimmen der blutbezogenen, individuellen anaeroben Schwelle einfacher und angenehmer. Alternativ oder zusätzlich kann mittels der erfindungsgemäßen Vorrichtung zur Datenverarbeitung für einen Probanden angegeben werden, wie sich seine Leistung zur speichelbezogenen, individuellen anaeroben Schwelle oder zur blutbezogenen, individuellen anaeroben Schwelle verhält. Anders gesagt gibt der Indikator für den Probanden an, über welche dominierenden Stoffwechselmechanismen er die Leistung bereitstellt. Auf diese Weise wird dem Probanden ermöglicht, seine Belastungen, d. h. die abgegebene Leistung, beispielsweise zu Trainingszwecken, präzise einzustellen.

Darüber hinaus wird die Aufgabe durch ein Computerprogramm gelöst, umfassend Befehle, die bei der Ausführung des Computerprogramms durch einen Computer diesen veranlassen, wenigstens eines der erfindungsgemäßen Verfahren auszuführen. Mittels des Computerprogramms kann also in einer ersten Alternative das erfindungsgemäße Verfahren zum Bestimmen einer blutbezogenen, individuellen anaeroben Schwelle eines Probanden ausgeführt werden. In einer zweiten Alternative kann mittels des Computerprogramms das erfindungsgemäße Verfahren zum Überwachen einer blutbezogenen, individuellen anaeroben Schwelle eines Probanden ausgeführt werden. In einer dritten Alternative können mittels des Computerprogramms sowohl das erfindungsgemäße Verfahren zum Bestimmen einer blutbezogenen, individuellen anaeroben Schwelle eines Probanden, als auch das erfindungsgemäße Verfahren zum Überwachen einer blutbezogenen, individuellen anaeroben Schwelle eines Probanden ausgeführt werden. Mittels des erfindungsgemäßen Computerprogramms kann also eine blutbezogene, individuelle anaerobe Schwelle eines Probanden präzise und zuverlässig erhalten werden, wobei gleichzeitig auf Blutentnahmen und -analysen verzichtet werden kann. Auf diese Weise wird das Bestimmen der blutbezogenen, individuellen anaeroben Schwelle einfacher und angenehmer. Alternativ oder zusätzlich kann mittels des erfindungsgemäßen Computerprogramms für einen Probanden angegeben werden, wie sich seine Leistung zur speichelbezogenen, individuellen anaeroben Schwelle oder zur blutbezogenen, individuellen anaeroben Schwelle verhält. Anders gesagt gibt der Indikator für den Probanden an, über welche dominierenden Stoffwechselmechanismen er die Leistung bereitstellt. Auf diese Weise wird dem Probanden ermöglicht, seine Belastungen, d. h. die abgegebene Leistung, beispielsweise zu Trainingszwecken, präzise einzustellen.

Auch wird die Aufgabe durch ein computerlesbares Medium gelöst, umfassend Befehle, die bei der Ausführung durch einen Computer diesen veranlassen, wenigstens eines der erfindungsgemäßen Verfahren auszuführen. Dabei kann das computerlesbare Medium auch als computerlesbares Speichermedium bezeichnet werden. Mittels des computerlesbaren Mediums kann also in einer ersten Alternative das erfindungsgemäße Verfahren zum Bestimmen einer blutbezogenen, individuellen anaeroben Schwelle eines Probanden ausgeführt werden. In einer zweiten Alternative kann mittels des computerlesbaren Mediums das erfindungsgemäße Verfahren zum Überwachen einer blutbezogenen, individuellen anaeroben Schwelle eines Probanden ausgeführt werden. In einer dritten Alternative können mittels des computerlesbaren Mediums sowohl das erfindungsgemäße Verfahren zum Bestimmen einer blutbezogenen, individuellen anaeroben Schwelle eines Probanden, als auch das erfindungsgemäße Verfahren zum Überwachen einer blutbezogenen, individuellen anaeroben Schwelle eines Probanden ausgeführt werden. Mittels des erfindungsgemäßen computerlesbaren Mediums kann also eine blutbezogene, individuelle anaerobe Schwelle eines Probanden präzise und zuverlässig erhalten werden, wobei gleichzeitig auf Blutentnahmen verzichtet werden kann. Auf diese Weise wird das Bestimmen der blutbezogenen, individuellen anaeroben Schwelle einfacher und angenehmer. Alternativ oder zusätzlich kann mittels des erfindungsgemäßen computerlesbaren Mediums für einen Probanden angegeben werden, wie sich seine Leistung zur speichelbezogenen, individuellen anaeroben Schwelle oder zur blutbezogenen, individuellen anaeroben Schwelle verhält. Anders gesagt gibt der Indikator für den Probanden an, über welche dominierenden Stoffwechselmechanismen er die Leistung bereitstellt. Auf diese Weise wird dem Probanden ermöglicht, seine Belastungen, d. h. die abgegebene Leistung, beispielsweise zu Trainingszwecken, präzise einzustellen.

In einem Beispiel ist das computerlesbare Medium oder computerlesbare Speichermedium als Bestandteil eines mobilen elektronischen Geräts ausgeführt. Beim mobilen elektronischen Gerät kann es sich beispielsweise um ein Mobiltelefon oder ein Tablet handeln. Ebenso kann das mobile elektronische Gerät ein Laptop-Computer sein. Auch ist es möglich, dass das mobile elektronische Gerät als Smart Watch ausgebildet ist. Ebenso ist es möglich, dass das computerlesbare Medium oder computerlesbare Speichermedium als Bestandteil eines stationären elektronischen Geräts ausgeführt ist, zum Beispiel als Bestandteil eines stationären Computers.

Ebenso wird die Aufgabe durch ein System zum Bestimmen einer blutbezogenen, individuellen anaeroben Schwelle eines Probanden gelöst. Das System umfasst eine erfindungsgemäße Vorrichtung zur Datenverarbeitung, umfassend Mittel zur Ausführung des erfindungsgemäßen Verfahrens zum Bestimmen einer blutbezogenen, individuellen anaeroben Schwelle eines Probanden. Ferner umfasst das System eine erste Sensoreinheit zum Erfassen wenigstens eines Speichellaktatkonzentrationswerts. Außerdem umfasst das System eine zweite Sensoreinheit zum Erfassen wenigstens eines Leistungswerts, welcher eine Leistung des Probanden beschreibt. Zusätzlich umfasst das System eine Zeiteinheit zum Bereitstellen eines Zeitwerts. Dabei sind die erste Sensoreinheit, die zweite Sensoreinheit und die Zeiteinheit kommunikationstechnisch mit der Vorrichtung zur Datenverarbeitung gekoppelt oder Bestandteile der Vorrichtung zur Datenverarbeitung. Wie bereits erwähnt, kann dabei die erste Sensoreinheit als intraorale Sensoreinheit oder als extraorale Sensoreinheit ausgeführt sein. Mittels des Systems zum Bestimmen einer blutbezogenen, individuellen anaeroben Schwelle eines Probanden kann also eine blutbezogene, individuelle anaerobe Schwelle eines Probanden präzise und zuverlässig erhalten werden, wobei gleichzeitig auf Blutentnahmen und -analysen verzichtet werden kann. Auf diese Weise wird das Bestimmen der individuellen anaeroben Schwelle einfacher und angenehmer.

Auch wird die Aufgabe durch ein System zum Überwachen einer blutbezogenen, individuellen anaeroben Schwelle eines Probanden gelöst. Das System umfasst eine Vorrichtung zur Datenverarbeitung, umfassend Mittel zur Ausführung des erfindungsgemäßen Verfahrens zum Überwachen einer blutbezogenen, individuellen anaeroben Schwelle eines Probanden. Ferner umfasst das System eine erste Sensoreinheit zum Erfassen wenigstens eines Speichellaktatkonzentrationswerts. Außerdem umfasst das System eine Zeiteinheit zum Bereitstellen eines Zeitwerts. Zusätzlich umfasst das System eine Ausgabeschnittstelle zum Ausgeben eines Indikators, welcher eine Beziehung wenigstens eines Speichellaktatkonzentrationswerts, der zu einem zugehörigen Erfassungszeitpunkt erfasst wurde, zur speichelbezogenen, individuellen anaeroben Schwelle oder zur blutbezogenen, individuellen anaeroben Schwelle beschreibt. Dabei sind die erste Sensoreinheit, die Zeiteinheit und die Ausgabeschnittstelle kommunikationstechnisch mit der Vorrichtung zur Datenverarbeitung gekoppelt oder Bestandteile der Vorrichtung zur Datenverarbeitung. Wie bereits erwähnt, kann dabei die erste Sensoreinheit als intraorale Sensoreinheit oder als extraorale Sensoreinheit ausgeführt sein. Somit kann mittels des Systems zum Überwachen einer blutbezogenen, individuellen anaeroben Schwelle eines Probanden für einen Probanden angegeben werden, wie sich seine Leistung zur speichelbezogenen, individuellen anaeroben Schwelle oder zur blutbezogenen, individuellen anaeroben Schwelle verhält. Anders gesagt gibt der Indikator für den Probanden an, über welche dominierenden Stoffwechselmechanismen er die Leistung bereitstellt. Auf diese Weise wird dem Probanden ermöglicht, seine Belastungen, d. h. die abgegebene Leistung, beispielsweise zu Trainingszwecken, präzise einzustellen.

Sowohl das erfindungsgemäße Verfahren zum Bestimmen einer blutbezogenen, individuellen anaeroben Schwelle eines Probanden, als auch das erfindungsgemäße Verfahren zum Überwachen einer blutbezogenen, individuellen anaeroben Schwelle, als auch das Verfahren zum Trainieren des Datenmodells können computerimplementierte Verfahren sein.

Alternativ oder zusätzlich zu den bereits erläuterten Verfahren kann basierend auf den ersten Daten, welche eine Leistung des Probanden beschreibende Leistungswerte, einen jeweils zugehörigen Erfassungszeitpunkt beschreibende Zeitwerte und jeweils zugehörige Speichellaktatkonzentrationswerte umfassen, auch ein Verfahren zum Ermitteln von Trainingsbereichen ausgeführt werden. Voraussetzung hierfür ist, dass implizit oder explizit basierend auf den ersten Daten eine blutbezogene, individuelle aerobe Schwelle des Probanden sowie eine blutbezogene, individuelle anaerobe Schwelle des Probanden ermittelt wurde. Dies wurde bereits erläutert. Es können somit zumindest drei Trainingsbereiche ermittelt werden, wobei ein erster Trainingsbereich dadurch charakterisiert ist, dass ein Blutlaktatkonzentrationswert unterhalb der blutbezogenen, individuellen aeroben Schwelle des Probanden liegt. Ein zweiter Trainingsbereich ist dadurch charakterisiert, dass ein Blutlaktatkonzentrationswert zwischen der blutbezogenen, individuellen aeroben Schwelle des Probanden und der blutbezogenen, individuellen anaeroben Schwelle des Probanden liegt. Ein dritter Trainingsbereich ist dadurch charakterisiert, dass ein Blutlaktatkonzentrationswert oberhalb der blutbezogenen, individuellen anaeroben Schwelle des Probanden liegt.

Basierend auf derartigen Trainingsbereichen kann ein Proband sein Training individualisiert und effizient steuern und/oder dokumentieren.

Alternativ zu diesen insgesamt drei Trainingsbereichen, die jeweils durch die blutbezogene, individuelle aerobe Schwelle des Probanden und die blutbezogene, individuelle anaerobe Schwelle des Probanden direkt charakterisiert oder definiert sind, lassen sich auch weitere Trainingsbereiche definieren, die sich beispielsweise anhand von Prozentwerten der der blutbezogenen, individuellen aeroben Schwelle des Probanden entsprechenden Leistung und/oder anhand von Prozentwerten der der blutbezogenen, individuellen anaeroben Schwelle des Probanden entsprechenden Leistung charakterisiert oder definiert werden können. Auch sind Mischformen möglich, d.h. es können zum Charakterisieren oder Definieren von Trainingsbereichen sowohl solche Prozentwerte als auch die blutbezogene, individuelle aerobe Schwelle des Probanden und/oder die blutbezogene, individuelle anaerobe Schwelle des Probanden als solche verwendet werden. Es lassen sich also die Trainingsbereiche anwendungs- oder bedarfsgerecht definieren oder charakterisieren. Dies trägt ebenfalls zu einer individualisierten und effizienten Steuerung und/oder Dokumentation des Trainings bei.

Insbesondere können in diesem Zusammenhang fünf Trainingsbereiche definiert werden, wobei ein erster Trainingsbereich durch eine Leistung von 70% oder weniger der der blutbezogenen, individuellen anaeroben Schwelle des Probanden entsprechenden Leistung charakterisiert oder definiert ist. Der erste Trainingsbereich kann auch als Kompensationsbereich bezeichnet werden. Ein zweiter Trainingsbereich kann durch eine Leistung oberhalb von 70% und bis inklusive 80% der der blutbezogenen, individuellen anaeroben Schwelle des Probanden entsprechenden Leistung charakterisiert oder definiert werden. Der zweite Trainingsbereich kann auch als erster Grundlagenausdauerbereich bezeichnet werden. Im zweiten Trainingsbereich wird die Bewegung oder Leistung des Probanden überwiegend durch sogenannte aerobe Energiegewinnung ermöglicht. Ein dritter Trainingsbereich kann durch eine Leistung oberhalb von 80% und bis inklusive 90% der der blutbezogenen, individuellen anaeroben Schwelle des Probanden entsprechenden Leistung charakterisiert oder definiert werden. Der dritte Trainingsbereich kann auch als zweiter Grundlagenausdauerbereich bezeichnet werden. Im dritten Trainingsbereich wird die Bewegung oder Leistung des Probanden weiterhin überwiegend durch sogenannte aerobe Energiegewinnung ermöglicht, ein kleiner Teil der Energiegewinnung erfolgt jedoch anaerob. Ein vierter Trainingsbereich kann durch eine Leistung oberhalb von 90% und bis inklusive 110% der der blutbezogenen, individuellen anaeroben Schwelle des Probanden entsprechenden Leistung charakterisiert oder definiert werden. Der vierte Trainingsbereich kann auch als Entwicklungsbereich bezeichnet werden. Im vierten Trainingsbereich wird die Bewegung oder Leistung des Probanden sowohl durch sogenannte aerobe Energiegewinnung als auch durch sogenannte anaerobe Energiegewinnung ermöglicht. Ein fünfter Trainingsbereich kann durch eine Leistung oberhalb von 110% der der blutbezogenen, individuellen anaeroben Schwelle des Probanden entsprechenden Leistung charakterisiert oder definiert werden. Der fünfte Trainingsbereich kann auch als Spitzenbereich bezeichnet werden. Im fünften Trainingsbereich wird die Bewegung oder Leistung des Probanden überwiegend durch sogenannte anaerobe Energiegewinnung ermöglicht. Im fünften Trainingsbereich kann der Proband die geforderte Leistung nur kurzzeitig abgeben. Anders gesagt kann der Proband im fünften Trainingsbereich der Belastung nur kurzzeitig widerstehen. Basierend auf derartigen Trainingsbereichen kann ein Proband sein Training individualisiert und effizient steuern und/oder dokumentieren. Aufgrund der hohen Anzahl an Trainingsbereichen kann dies vergleichsweise genau und detailliert erfolgen.

Es versteht sich, dass die Trainingsbereiche auch auf andere Art und Weise definiert sein können. Somit kann die Anzahl der Trainingsbereiche sowie deren Definition oder Charakterisierung so gewählt werden, dass dies für die gewünschte Steuerung und/oder Dokumentation des Trainings passend ist.

Es versteht sich, dass für die Ermittlung der Trainingsbereiche ein Datenmodell verwendet werden kann, insbesondere ein entsprechend trainiertes Datenmodell. In diesem Zusammenhang ist es auch möglich, dass die Trainingsbereiche direkt ermittelt werden, d.h. ohne, dass zuvor explizit und separat die blutbezogene, individuelle aerobe Schwelle des Probanden sowie die blutbezogene, individuelle anaerobe Schwelle des Probanden ermittelt wurde. Ein derartiges Datenmodell kann ebenfalls mittels eines Verfahrens zum Trainieren eines Datenmodells trainiert werden. Das Datenmodell wird dabei mittels eines annotierten Datensatzes trainiert, welcher mehrere Trainingsdatenelemente umfasst, welche eine Leistung beschreibende Leistungswerte, einen jeweils zugehörigen Erfassungszeitpunkt beschreibende Zeitwerte und jeweils zugehörige Speichellaktatkonzentrationswerte umfassen, wobei die Trainingsdatenelemente mit zugehörigen Trainingsbereichen annotiert sind. Die Trainingsdatenelemente entsprechen also inhaltlich den zuvor beschriebenen ersten Daten. Mittels eines derart trainierten Datenmodells lassen sich, wie bereits erläutert, einfach und zuverlässig anhand von ersten Daten, welche eine Leistung des Probanden beschreibende Leistungswerte, einen jeweils zugehörigen Erfassungszeitpunkt beschreibende Zeitwerte und jeweils zugehörige Speichellaktatkonzentrationswerte umfassen, Trainingsbereiche bestimmen, ohne dass dafür Blutproben genommen und/oder analysiert werden müssen. Mit anderen Worten erlaubt ein derartig trainiertes Datenmodell, lediglich anhand von Messungen im Speichel Trainingsbereiche zu bestimmen.

Die Erfindung wird nachstehend anhand verschiedener Ausführungsbeispiele erläutert, die in den beigefügten Zeichnungen gezeigt sind. Es zeigen:
- Figur 1: einen Probanden, der ein erfindungsgemäßes System zum Bestimmen einer blutbezogenen, individuellen anaeroben Schwelle nutzt, wobei mittels des Systems ein erfindungsgemäßes Verfahren zum Bestimmen einer blutbezogenen, individuellen anaeroben Schwelle des Probanden ausgeführt wird,
- Figur 2: eine grafische Repräsentation von ersten Daten, die beim Ausführen des erfindungsgemäßen Verfahrens zum Bestimmen einer blutbezogenen, individuellen anaeroben Schwelle des Probanden verwendet werden,
- Figur 3: eine Illustration eines Verfahrensschritts zum Bestimmen einer speichelbezogenen, individuellen aeroben Schwelle,
- Figur 4: eine grafische Repräsentation eines Datenmodells, das ein Regressionsmodell umfasst, welches einen Zusammenhang zwischen einer blutbezogenen, individuellen anaeroben Schwelle und einer speichelbezogenen, individuellen anaeroben Schwelle beschreibt,
- Figur 5: eine grafische Repräsentation eines Datenmodells, das ein Regressionsmodell umfasst, welches einen Zusammenhang zwischen einer blutbezogenen, individuellen aeroben Schwelle und einer speichelbezogenen, individuellen aeroben Schwelle beschreibt,
- Figur 6: eine grafische Repräsentation eines Datenmodells, das ein Regressionsmodell umfasst, welches einen Zusammenhang zwischen einem maximalen Blutlaktatkonzentrationswert und einem maximalen Speichellaktatkonzentrationswert beschreibt,
- Figur 7: eine grafische Repräsentation eines Datenmodells, das ein trainiertes Datenmodell umfasst,
- Figur 8: eine Illustration eines Trainings des Datenmodells aus Figur 7,
- Figur 9: einen Probanden, der ein erfindungsgemäßes System zum Überwachen einer blutbezogenen, individuellen anaeroben Schwelle nutzt, wobei mittels des Systems ein erfindungsgemäßes Verfahren zum Überwachen einer blutbezogenen, individuellen anaeroben Schwelle des Probanden ausgeführt wird, und
- Figur 10: eine Illustration von verschiedenen Trainingsbereichen, die in Abhängigkeit einer blutbezogenen, individuellen aeroben Schwelle und einer blutbezogenen, individuellen anaeroben Schwelle definiert sind.

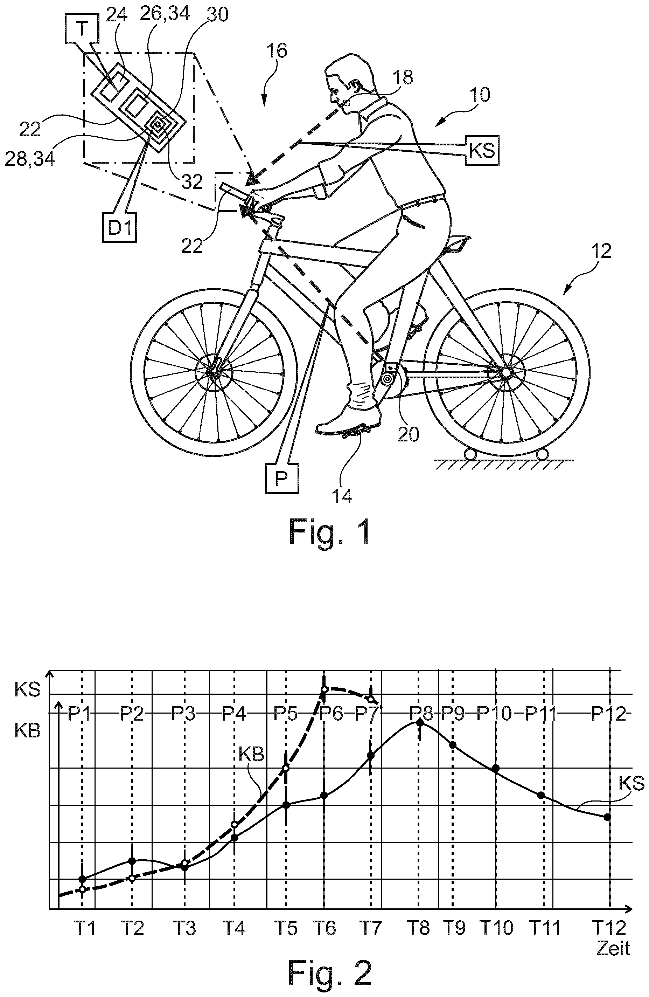

Figur 1 zeigt einen Probanden 10, der einen Leistungsstufentest absolviert.

Dabei sitzt der Proband 10 auf einem Fahrradergometer 12 und tritt in die Pedale 14, wobei ein Widerstand der Pedale 14, der dem Treten des Probanden 10 entgegengesetzt wird, einstellbar ist.

Somit wird im Zuge des Leistungsstufentests der Widerstand stufenweise erhöht. Genauer gesagt startet der Test bei einem vorgegebenen, vergleichsweise niedrigen Leistungsniveau des Probanden 10, z. B. 70 Watt, das einem vorgegebenen Widerstand der Pedale 14 entspricht. Nach Ablauf eines vorgegebenen Zeitintervalls, z. B. 3 Minuten, wird der Widerstand der Pedale 14 stets um ein vorgegebenes Inkrement erhöht, z. B. um 30 Watt. Der Proband 10 muss also eine um ein entsprechendes Leistungsinkrement erhöhte Leistung abgeben.

Der Widerstand der Pedale 14, d. h. die Leistung des Probanden 10, wird dabei so lange stufenweise um jeweils ein Leistungsinkrement erhöht, bis der Proband 10 vor Erschöpfung aufgibt. Ab diesem Zeitpunkt kann der Proband entweder ruhen oder gegen einen vergleichsweise geringen Widerstand, z. B. 50 Watt oder 70 Watt, die Pedale 14 weiter treten. Es versteht sich dabei, dass zum Schutze des Probanden 10 der Leistungsstufentest auch jederzeit aus medizinischen Gründen abgebrochen werden kann. Dies stellt jedoch die Ausnahme dar.

Der Proband 10 nutzt im Zusammenhang mit dem Leistungsstufentest ein System 16 zum Bestimmen seiner blutbezogenen, individuellen anaeroben Schwelle ANS.

Dieses System 16 umfasst eine erste Sensoreinheit 18 zum Erfassen eines Speichellaktatkonzentrationswerts KS. In der dargestellten Ausführungsform ist die erste Sensoreinheit 18 als intraorale Sensoreinheit, d.h. als Intraoralsensor, ausgeführt. Das bedeutet, dass der Proband 10 die erste Sensoreinheit 18 in seinem Mund trägt.

Mittels der ersten Sensoreinheit 18 kann kontinuierlich oder quasi-kontinuierlich ein Speichellaktatkonzentrationswert KS gemessen werden.

Das System 16 umfasst darüber hinaus eine zweite Sensoreinheit 20. Die zweite Sensoreinheit 20 ist zum Erfassen eines Leistungswerts P ausgebildet, der eine Leistung des Probanden 10 beschreibt. Im dargestellten Ausführungsbeispiel ist die zweite Sensoreinheit 20 am Fahrradergometer 12 angebracht, sodass mittels der zweiten Sensoreinheit 20 eine Leistung des Probanden 10 gemessen werden kann, die der Proband 10 beim Treten in die Pedale 14 abgibt.

Darüber hinaus umfasst das System 16 eine Vorrichtung 22 zur Datenverarbeitung.

Die Vorrichtung 22 zur Datenverarbeitung ist in der dargestellten Ausführungsform als mobiles elektronisches Gerät ausgeführt, das am Lenker des Fahrradergometers 12 befestigt ist. Beim mobilen elektronischen Gerät kann es sich beispielsweise um ein Mobiltelefon oder ein Tablet handeln. Auch ist es möglich, dass das mobile elektronische Gerät als Smart Watch ausgebildet ist. Ebenso ist es möglich, dass die Vorrichtung 22 zur Datenverarbeitung als stationäres elektronisches Gerät, zum Beispiel als stationärer Computer, oder Laptop ausgebildet ist.

Dabei ist die erste Sensoreinheit 18 kommunikationstechnisch mit der Vorrichtung 22 zur Datenverarbeitung gekoppelt. Im dargestellten Ausführungsbeispiel handelt es sich bei der kommunikationstechnischen Kopplung um eine drahtlose Kopplung, zum Beispiel Bluetooth. Dies ist durch eine gestrichelte Linie illustriert.

Ebenso ist die zweite Sensoreinheit 20 kommunikationstechnisch mit der Vorrichtung 22 zur Datenverarbeitung gekoppelt. Im dargestellten Ausführungsbeispiel handelt es sich auch bei dieser kommunikationstechnischen Kopplung um eine drahtlose Kopplung, zum Beispiel Bluetooth. Dies ist ebenfalls durch eine gestrichelte Linie illustriert.

Das System 16 umfasst ferner eine Zeiteinheit 24, die zum Bereitstellen eines Zeitwerts ausgebildet ist. In der dargestellten Ausführungsform ist die Zeiteinheit 24 als Bestandteil der Vorrichtung 22 zur Datenverarbeitung ausgeführt und daher mit allen anderen Komponenten der Vorrichtung 22 zur Datenverarbeitung kommunikationstechnisch gekoppelt. Alternativ wäre es auch denkbar, die Zeiteinheit 24 als von der Vorrichtung 22 zur Datenverarbeitung separate Einheit vorzusehen. In dieser Alternative muss die Zeiteinheit 24 kommunikationstechnisch mit der Vorrichtung 22 zur Datenverarbeitung gekoppelt sein. Dies kann wieder über Bluetooth erfolgen.

Die Vorrichtung 22 zur Datenverarbeitung umfasst ferner eine Datenverarbeitungseinheit 26 und eine Datenspeichereinheit 28

Dabei umfasst die Datenspeichereinheit 28 ein computerlesbares Medium 30.

Auf dem computerlesbaren Medium 30 ist ein Computerprogramm 32 hinterlegt, welches Befehle umfasst, die bei der Ausführung des Computerprogramms 32 durch die Datenverarbeitungseinheit 26, allgemeiner gesprochen durch einen Computer, diesen veranlassen, ein Verfahren zum Bestimmen einer blutbezogenen, individuellen anaeroben Schwelle ANS eines Probanden 10 auszuführen.

Somit umfasst auch das computerlesbare Medium 30 Befehle, die bei der Ausführung durch die Datenverarbeitungseinheit 26, allgemeiner gesprochen durch einen Computer, diesen veranlassen, ein Verfahren zum Bestimmen einer blutbezogenen, individuellen anaeroben Schwelle ANS eines Probanden 10 auszuführen.

Die Datenverarbeitungseinheit 26 und die Datenspeichereinheit 28 stellen somit Mittel 34 zur Ausführung eines Verfahrens zum Bestimmen einer blutbezogenen, individuellen anaeroben Schwelle ANS eines Probanden 10 dar.

Im Folgenden wird das Verfahren zum Bestimmen einer blutbezogenen, individuellen anaeroben Schwelle ANS näher erläutert.

In einem ersten Schritt S11 werden erste Daten D1 erhalten, welche eine Leistung des Probanden 10 beschreibende Leistungswerte P, einen jeweils zugehörigen Erfassungszeitpunkt beschreibende Zeitwerte T und jeweils zugehörige Speichellaktatkonzentrationswerte KS umfassen.

Die ersten Daten D1 werden dabei beim Ausführen des Leistungsstufentest ermittelt, wobei die die Leistung des Probanden 10 beschreibende Leistungswerte P mittels der zweiten Sensoreinheit 20 ermittelt werden und die Speichellaktatkonzentrationswerte KS mittels der ersten Sensoreinheit 18 ermittelt werden. Nachdem sowohl die erste Sensoreinheit 18 als auch die zweite Sensoreinheit 20 kommunikationstechnisch mit der Vorrichtung 22 zur Datenverarbeitung gekoppelt sind, welche die Zeiteinheit 24 umfasst, kann jedem Leistungswerte P und jedem Laktatkonzentrationswert KS ein Zeitwert T, der einen Erfassungszeitpunkt beschreibt, zugeordnet werden.

Folglich werden die ersten Daten D1 an der Vorrichtung 22 zur Datenverarbeitung erhalten und/oder liegen an der Vorrichtung 22 zur Datenverarbeitung vor.

Die ersten Daten D1 sind in Figur 2 grafisch repräsentiert, wobei auf der horizontalen Achse die Zeit aufgetragen ist und auf einer ersten vertikalen Achse die Speichellaktatkonzentration KS.

Figur 2 illustriert in diesem Zusammenhang sowohl einige spezifische Messwerte für die Speichellaktatkonzentration KS als auch eine Kurve, mittels der die Messwerte interpoliert sind. Es versteht sich, dass sowohl die Messwerte also auch die Kurve als rein illustrativ zu verstehen sind.

Nachdem die Leistung sich in vorgegebenen Zeitintervallen, z. B. 3 Minuten, um jeweils eine vorgegebene Leistungsstufe, z. B. 30 Watt, erhöht, lässt sich ferner jedem Speichellaktatkonzentrationswert KS ein Leistungswert P zuordnen.

Nachdem die Leistungswerte für jeden Probanden 10 unterschiedlich sind, sind diese in der Figur 2 lediglich mit P1 bis P12 bezeichnet. Dabei hat im vorliegenden Beispiel der Proband 10 beim Leistungswert P6 seine maximale Leistung erreicht. Das bedeutet, dass die Leistungswerte von P1 bis P6 kontinuierlich ansteigen. Die Leistungswerte P7 bis P12 sind vergleichsweise klein, zum Beispiel im Bereich des Leistungswerts P1. Auch die Leistungswerte P sind dabei als rein illustrativ zu verstehen.

Es wird bemerkt, dass in einem Fall, in dem der Proband 10 den höchsten Leistungswert P nicht während des gesamten zugeordneten Zeitintervalls erbringen kann, der höchste Leistungswert P durch Interpolation ermittelt werden kann. Beispielsweise kann dabei zur Ermittlung des höchsten Leistungswerts P derjenige Prozentsatz der festen Leistungsstufe zum vorhergehenden Leistungswert P addiert werden, der dem Anteil des Zeitintervalls entspricht, über den der Proband 10 die Leistung erbringen konnte. Wenn der Proband also bei einer Leistungsstufe von 30 Watt den höchsten Leistungswerts während 80 % des vordefinierten Zeitintervalls, zum Beispiel in Höhe von 3 Minuten, halten konnte, wird die höchste Leistung dadurch ermittelt, dass 80 % von 30 Watt zur vorhergehenden Leistungsstufe addiert werden.

Es wird dabei insbesondere anhand der Darstellung in Figur 2 deutlich, dass die ersten Daten D1 über einen maximalen Speichellaktatkonzentrationswert KS hinausgehen. Ferner wird deutlich, dass der maximale Speichellaktatkonzentrationswert KS zeitlich erst nach dem Erreichen des maximalen Leistungswerts P auftritt.

Zum besseren Verständnis ist in Figur 2 mittels einer gestrichelten Linie zudem ein exemplarischer Verlauf einer Blutlaktatkonzentration KB eingezeichnet. Die Blutlaktatkonzentration KB ist dabei entlang einer zweiten vertikalen Achse aufgetragen, die von der ersten vertikalen Achse verschieden ist. Dies dient lediglich dem besseren Verständnis. Im dargestellten Ausführungsbeispiel wird ausdrücklich keine Blutlaktatkonzentration direkt erfasst, sondern lediglich anhand der sensorisch erfassten Speichellaktatkonzentration ermittelt, wie im Folgenden noch im Detail erläutert werden wird.

In einem zweiten Schritt S12 wird zunächst die speichelbezogene, individuelle aerobe Schwelle SAES bestimmt.

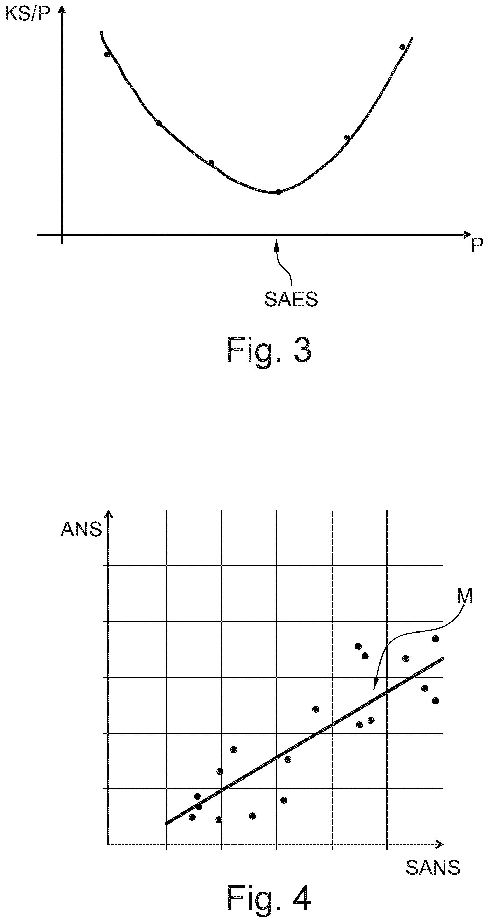

Hierzu wird basierend auf den ersten Daten D1 ein Quotient aus den erfassten Speichellaktatkonzentrationswerten KS und den jeweils zugeordneten Leistungswerten P ermittelt und virtuell oder tatsächlich über die Leistung aufgetragen. Derjenige Leistungswert P, bei dem der Quotient minimal ist, ist die speichelbezogene, individuelle aerobe Schwelle SAES (siehe Figur 3).

In einer alternativen Ausprägung des zweiten Schrittes S12 kann die speichelbezogene, individuelle aerobe Schwelle SAES auch durch Auswerten einer zeitlichen Änderungsrate der Speichellaktatkonzentrationswerte KS aus den ersten Daten D1 bestimmt werden.

Wie bereits erwähnt, ist die aerobe Schwelle durch denjenigen kleinsten Leistungswert gebildet, bei dem eine Laktatkonzentration über eine Ruhe-Laktatkonzentration ansteigt. Mit anderen Worten kann die speichelbezogene aerobe Schwelle SAES als diejenige Leistung ermittelt werden, bei der der Gradient der Speichellaktatkonzentration KS erstmals wesentlich von Null abweicht. Dabei kann eine wesentliche Abweichung dadurch definiert sein, dass der Gradient um mindestens einen vorgegebenen Wert von Null abweicht. Hierfür können die Laktatkonzentrationswerte KS aus den ersten Daten D1 verwendet werden, die zum Bestimmen eines Gradienten auch interpoliert werden können (siehe Figur 2).

In einem nachfolgenden dritten Schritt S13 wird die speichelbezogene, individuelle anaerobe Schwelle SANS ermittelt.

Hierfür wird das für sich genommen bekannte D-max-Verfahren verwendet. In diesem Zusammenhang wird eine Gerade erstellt, die sich von derjenigen Laktatkonzentration KS, die an der im zweiten Schritt ermittelten, speichelbezogenen, individuellen aeroben Schwelle SAES vorliegt, zu einer maximalen Speichellaktatkonzentration KS erstreckt.

Sodann wird ein Speichellaktatkonzentrationswert KS ermittelt, der einen maximalen Abstand von dieser Geraden aufweist. Die speichelbezogene, individuelle anaerobe Schwelle SANS wird dann als derjenige Leistungswert P definiert, der dem Speichellaktatkonzentrationswert KS mit dem größten Abstand zugeordnet ist.

Dies kann anhand der Figur 2 grafisch erfolgen. Alternativ kann dies von der Vorrichtung 22 zur Datenverarbeitung ausgeführt werden.

In einer alternativen Ausprägung des dritten Schrittes S13 wird die Gerade anstatt zwischen demjenigen Speichellaktatkonzentrationswert KS, der an der aeroben Schwelle SAES vorliegt und dem maximalen Speichellaktatkonzentrationswert KS zwischen dem minimalen Speichellaktatkonzentrationswert KS und dem maximalen Speichellaktatkonzentrationswert KS erstellt. Es versteht sich, dass es im Falle dieser alternativen Ausprägung des dritten Schrittes S13 nicht notwendig ist, im zweiten Schritt S12 die speichelbezogene, individuelle aerobe Schwelle SAES zu bestimmen. Auch dies kann grafisch anhand der Figur 2 erfolgen oder von der Vorrichtung 22 zur Datenverarbeitung ausgeführt werden.

In einer weiteren alternativen Ausprägung des dritten Schrittes S13 wird die speichelbezogene, individuelle anaerobe Schwelle SANS durch ein Auswerten einer zeitlichen Änderungsrate der Speichellaktatkonzentrationswerte KS erhalten. Wie bereits erläutert, ist die speichelbezogene, individuelle anaerobe Schwelle SANS dadurch definiert, dass Laktat derart schnell generiert wird, dass es durch die körpereigenen Abbauprozesse nicht mehr im Fließgleichgewicht gehalten werden kann. Das bedeutet, dass an der anaeroben Schwelle SANS ein Gradient der Speichellaktatkonzentration KS ausgehend von einem im Wesentlichen konstanten Wert beginnt zu steigen. Das lässt sich entweder anhand einer virtuellen oder tatsächlichen Kurve, die Laktatkonzentrationen KS beschreibt, ermitteln. Auch dies kann basierend auf Figur 2 geschehen.

Nach dem Ausführen des dritten Schrittes S13 ist somit die speichelbezogene, individuelle anaerobe Schwelle SANS des Probanden 10 bekannt.

In einem vierten Schritt S 14 wird die speichelbezogene, individuelle anaerobe Schwelle SANS des Probanden 10 in eine blutbezogene, individuelle anaerobe Schwelle ANS des Probanden 10 umgerechnet.

Hierzu wird ein Datenmodell M verwendet, das in der vorliegenden Ausführungsform ein Regressionsmodell ist, das auf linearer Regression beruht. Das Datenmodell M beschreibt einen Zusammenhang zwischen der blutbezogenen, individuellen anaeroben Schwelle ANS und einer speichelbezogenen, individuellen anaeroben Schwelle SANS in Form einer Geraden, kann also durch eine Geradengleichung beschrieben werden (siehe Figur 4).

Das vorliegende lineare Regressionsmodell wurde dadurch erstellt, dass bei einer Mehrzahl an Probanden sowohl die blutbezogene, individuelle anaerobe Schwelle ANS als auch die speichelbezogene, individuelle anaerobe Schwelle SANS bestimmt wurde. Hierfür kann beispielsweise der hier verwendete Leistungsstufentest verwendet werden. Die Bestimmung der blutbezogenen, individuellen anaeroben Schwelle ANS kann auf tatsächlichen Blutproben basieren. Folglich kann basierend auf der Mehrzahl an Probanden der Zusammenhang zwischen der blutbezogenen, individuellen anaeroben Schwelle ANS und der speichelbezogenen, individuellen anaeroben Schwelle SANS bestimmt werden. Dieser Zusammenhang kann dann in Form des Regressionsmodells für das Bestimmen der blutbezogenen, individuellen anaeroben Schwelle ANS bei weiteren Probanden, z. B. dem hier beschriebenen Probanden 10, verwendet werden, wofür dann keine Untersuchung von Blutproben mehr nötig ist.

Das lineare Regressionsmodell ist in Figur 4 illustriert, wobei exemplarisch eine Mehrzahl an Messpunkten dargestellt sind, die jeweils einem der Mehrzahl an Probanden zuzuordnen sind, die beim Erstellen des Datenmodells M mitgewirkt haben. Die aus diesen Messpunkten resultierende Gerade, die das eigentliche Datenmodell M darstellt, ist ebenso in Figur 4 gezeigt.

Im Zuge des vierten Schrittes S 14 kann somit die im dritten Schritt S13 ermittelte speichelbezogene, individuelle anaerobe Schwelle SANS ins Datenmodell M eingegeben werden. Das Datenmodell M gibt dann die blutbezogene, individuelle anaerobe Schwelle ANS aus.

Insgesamt wird somit durch Anwendung des Verfahrens zum Bestimmen einer blutbezogenen, individuellen anaeroben Schwelle ANS eines Probanden 10, die blutbezogene, individuelle anaerobe Schwelle ANS basierend auf den ersten Daten D1 und unter Nutzung eines Datenmodells M bestimmt. Es sind keinerlei Blutentnahmen oder Blutanalysen notwendig.

Das Verfahren zum Bestimmen einer blutbezogenen, individuellen anaeroben Schwelle eines Probanden kann auch gemäß einer ersten Variante ausgestaltet sein. Im Folgenden wird dabei lediglich auf die Unterschiede zur bereits erläuterten Basis-Variante des Verfahrens eingegangen.

Der wesentliche Unterschied besteht darin, dass das verwendete Datenmodell M, das wieder ein Regressionsmodell umfasst, einen Zusammenhang zwischen der blutbezogenen, individuellen aeroben Schwelle AES und der speichelbezogenen, individuellen aeroben Schwelle SAES beschreibt.

Dabei bleiben der erste Schritt S11 und der zweite Schritt S12 gegenüber der bereits erläuterten Basis-Variante gleich, wobei als Ergebnis des zweiten Schrittes S12 die speichelbezogene, individuelle aerobe Schwelle SAES ermittelt wird.

Nun wird anhand des Datenmodells M, das einen Zusammenhang zwischen der blutbezogenen, individuellen aeroben Schwelle AES und der speichelbezogenen, individuellen aeroben Schwelle SAES beschreibt, die blutbezogene, individuelle aerobe Schwelle AES des Probanden 10 ermittelt.

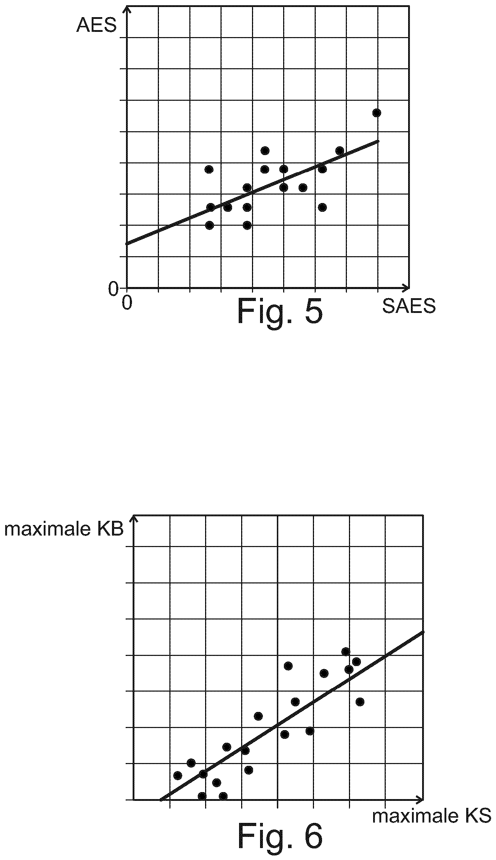

Dieses Datenmodell M kann ein lineares Regressionsmodell sein, das in Figur 5 illustriert ist, wobei exemplarisch eine Mehrzahl an Messpunkten dargestellt sind, die jeweils einem der Mehrzahl an Probanden zuzuordnen sind, die beim Erstellen des Datenmodells M mitgewirkt haben. Die aus diesen Messpunkten resultierende Gerade, die das eigentliche Datenmodell M darstellt, ist ebenso in Figur 5 gezeigt.

Darüber hinaus wird ein weiteres Datenmodell verwendet, das ebenfalls ein Regressionsmodell, insbesondere ein lineares Regressionsmodell, umfasst. Dieses Datenmodell beschreibt einen Zusammenhang zwischen einem maximalen Speichellaktatkonzentrationswert KS und einem maximalen Blutlaktatkonzentrationswert KB und ist in Figur 6 illustriert. Dabei ist wieder exemplarisch eine Mehrzahl an Messpunkten dargestellt, die jeweils einem der Mehrzahl an Probanden zuzuordnen sind, die beim Erstellen des Datenmodells M mitgewirkt haben. Die aus diesen Messpunkten resultierende Gerade, die das eigentliche Datenmodell M darstellt, ist ebenso in Figur 6 gezeigt.

Unter Nutzung dieses Datenmodells lässt sich also anhand des maximalen Speichellaktatkonzentrationswerts KS aus den ersten Daten D1, ein maximaler Blutlaktatkonzentrationswert KB ermitteln.

Nun kann wieder das für sich genommen bekannte D-max-Verfahren angewendet werden. Dieses Mal wird allerdings die Gerade zwischen dem Laktatkonzentrationswert der blutbezogenen, individuellen aeroben Schwelle AES und dem maximalen Blutlaktatkonzentrationswert KB gezogen.

Ferner müssen zum Ausführen des D-max-Verfahrens Speichellaktatkonzentrationswerte KS, die zwischen der speichelbezogenen, individuellen aeroben Schwelle SAES und dem maximalen Speichellaktatkonzentrationswert KS liegen, in Blutlaktatkonzentrationswerte KB umgerechnet werden. Dabei können die Speichellaktatkonzentrationswerte KS einzeln in Blutlaktatkonzentrationswerte KB umgerechnet werden. Optional kann dann eine Kurve durch die Blutlaktatkonzentrationswerte KB gelegt werden, z. B. im Zuge eines Fittings. Alternativ kann eine Kurve durch die Speichellaktatkonzentrationswerte KS gelegt werden, z. B. im Zuge eines Fittings, und sodann die Kurve, die die Speichellaktatkonzentrationswerte KS beschreibt, in eine Kurve umgerechnet werden, die Blutlaktatkonzentrationswerte KB beschreibt.

Das Verfahren zum Bestimmen einer blutbezogenen, individuellen anaeroben Schwelle ANS eines Probanden 10 kann auch gemäß einer zweiten Variante ausgestaltet sein. Im Folgenden wird dabei lediglich auf die Unterschiede zu den bereits erläuterten Varianten des Verfahrens eingegangen.

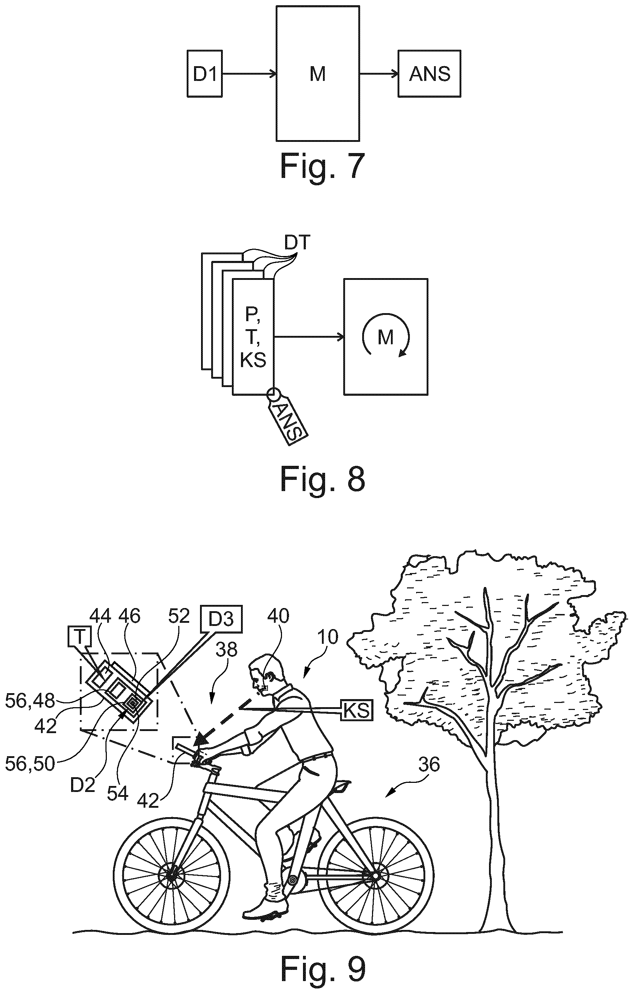

Der wesentliche Unterschied besteht darin, dass in der zweiten Variante das Datenmodell M ein trainiertes Datenmodell M umfasst. Dieses trainierte Datenmodell M ist dazu ausgebildet, die ersten Daten D1 als Eingangsdaten zu verwenden und direkt die blutbezogene, individuelle anaerobe Schwelle ANS auszugeben (siehe Illustration in Figur 7). Das bedeutet, dass das trainierte Datenmodell M direkt anschließend an den ersten Schritt S11 verwendet werden kann und die oben beschriebenen Schritte S12, S13 und S 14 entfallen können.

Das trainierte Datenmodell M ist dabei beispielsweise als künstliches neuronales Netz ausgebildet.

Das Training des trainierten Datenmodells M kann dabei wie folgt gestaltet sein (siehe auch Figur 8).

Zum Training des Datenmodells M kann dabei ein annotierter Datensatz verwendet werden, der mehrere Trainingsdatenelemente DT umfasst. Jedes der Trainingsdatenelemente DT umfasst dabei einen Leistungswert P, der eine Leistung beschreibt, einen Zeitwert T, der einen zugehörigen Erfassungszeitpunkts beschreibt und einen Speichellaktatkonzentrationswert KS. Diese Trainingsdatenelemente DT sind mit zugehörigen blutbezogenen, individuellen anaeroben Schwellen ANS annotiert.

Folglich kann das Datenmodell M im Rahmen des Trainings lernen, blutbezogene, individuelle anaerobe Schwellen ANS basierend auf ersten Daten D1 zu ermitteln. Wie bereits erläutert betreffen dabei die ersten Daten D1 Daten, welche eine Leistung des Probanden 10 beschreibende Leistungswerte P, einen jeweils zugehörigen Erfassungszeitpunkt beschreibende Zeitwerte T und jeweils zugehörige Speichellaktatkonzentrationswerte KS umfassen.

Figur 9 zeigt wieder den Probanden 10, der nun jedoch keinen Leistungsstufentest unter Nutzung eines Fahrradergometer 12 ausführt, sondern mit einem Fahrrad 36 im Freien unterwegs ist.

Der Proband 10 nutzt dabei ein System 38 zum Überwachen seiner blutbezogenen, individuellen anaeroben Schwelle ANS.

Dieses System 38 umfasst eine erste Sensoreinheit 40 zum Erfassen eines Speichellaktatkonzentrationswerts KS. In der dargestellten Ausführungsform ist die erste Sensoreinheit 40 als intraorale Sensoreinheit ausgeführt. Das bedeutet, dass der Proband 10 die erste Sensoreinheit 18 in seinem Mund trägt.

Mittels der ersten Sensoreinheit 40 kann kontinuierlich oder quasi-kontinuierlich ein Speichellaktatkonzentrationswert KS gemessen werden.

Dabei können die erste Sensoreinheit 18, die im Zusammenhang mit dem Verfahren zum Bestimmen der blutbezogenen, individuellen anaeroben Schwelle ANS verwendet wurde, und die erste Sensoreinheit 40 identisch ausgebildet oder sogar tatsächlich identisch sein. Im letztgenannten Fall kann also dieselbe erste Sensoreinheit 18, 40 sowohl ein Bestandteil des Systems 16 zum Bestimmen einer blutbezogenen, individuellen anaeroben Schwelle ANS sein als auch Bestandteil eines Systems 38 zum Überwachen einer blutbezogenen, individuellen anaeroben Schwelle ANS.

Darüber hinaus umfasst das System 38 eine Vorrichtung 42 zur Datenverarbeitung.

Die Vorrichtung 42 zur Datenverarbeitung ist in der dargestellten Ausführungsform als mobiles elektronisches Gerät ausgeführt, das am Lenker des Fahrrads 36 befestigt ist. Beim mobilen elektronischen Gerät kann es sich beispielsweise um ein Mobiltelefon oder ein Tablet handeln. Auch ist es möglich, dass das mobile elektronische Gerät als Smart Watch ausgebildet ist.

Dabei ist die erste Sensoreinheit 40 kommunikationstechnisch mit der Vorrichtung 42 zur Datenverarbeitung gekoppelt. Im dargestellten Ausführungsbeispiel handelt es sich bei der kommunikationstechnischen Kopplung um eine drahtlose Kopplung, zum Beispiel Bluetooth. Dies ist durch eine gestrichelte Linie illustriert.

Das System 38 umfasst ferner eine Zeiteinheit 44, die zum Bereitstellen eines Zeitwerts T ausgebildet ist. In der dargestellten Ausführungsform ist die Zeiteinheit 44 als Bestandteil der Vorrichtung 42 zur Datenverarbeitung ausgeführt und daher mit allen anderen Komponenten der Vorrichtung 42 zur Datenverarbeitung kommunikationstechnisch gekoppelt. Alternativ wäre es auch denkbar, die Zeiteinheit 44 als von der Vorrichtung 42 zur Datenverarbeitung separate Einheit vorzusehen. In dieser Alternative muss die Zeiteinheit 44 kommunikationstechnisch mit der Vorrichtung 42 zur Datenverarbeitung gekoppelt sein. Dies kann wieder über Bluetooth erfolgen.

Auch umfasst das System 38 eine Ausgabeschnittstelle 46 zum Ausgeben eines Indikators, welcher eine Beziehung wenigstens eines Speichellaktatkonzentrationswerts KS, der zu einem zugehörigen Erfassungszeitpunkt T erfasst wurde, zur speichelbezogenen, individuellen anaeroben Schwelle SANS oder zur blutbezogenen, individuellen anaeroben Schwelle ANS beschreibt.

In der dargestellten Ausführungsform ist die Ausgabeschnittstelle 46 durch eine Bildschirmeinheit des die Vorrichtung 42 zur Datenverarbeitung bildenden elektronischen Geräts gebildet. Die Ausgabeschnittstelle 46 ist also beispielsweise als Bildschirmeinheit eines Mobiltelefons, Tablets oder einer Smart Watch gebildet.

Folglich kann die Beziehung wenigstens eines Speichellaktatkonzentrationswerts KS, der zu einem zugehörigen Erfassungszeitpunkt T erfasst wurde, zur speichelbezogenen, individuellen anaeroben Schwelle SANS oder zur blutbezogenen, individuellen anaeroben Schwelle ANS grafisch dargestellt, d.h. für den Probanden 10 ausgegeben, werden.

Die Ausgabeschnittstelle 46 ist somit auch kommunikationstechnisch mit den übrigen Komponenten der Vorrichtung 42 zur Datenverarbeitung gekoppelt. Allgemeiner gesprochen ist die Ausgabeschnittstelle 46 mit der Vorrichtung 42 zur Datenverarbeitung gekoppelt.

Die Vorrichtung 42 zur Datenverarbeitung umfasst ferner eine Datenverarbeitungseinheit 48 und eine Datenspeichereinheit 50.

Dabei umfasst die Datenspeichereinheit 50 ein computerlesbares Medium 52.

Auf dem computerlesbaren Medium 52 ist ein Computerprogramm 54 hinterlegt, welches Befehle umfasst, die bei der Ausführung des Computerprogramms 54 durch die Datenverarbeitungseinheit 48, allgemeiner gesprochen durch einen Computer, diesen veranlassen, ein Verfahren zum Überwachen einer blutbezogenen, individuellen anaeroben Schwelle ANS eines Probanden 10 auszuführen.

Somit umfasst auch das computerlesbare Medium 52 Befehle, die bei der Ausführung durch die Datenverarbeitungseinheit 48, allgemeiner gesprochen durch einen Computer, diesen veranlassen, ein Verfahren zum Überwachen einer blutbezogenen, individuellen anaeroben Schwelle ANS eines Probanden 10 auszuführen.

Die Datenverarbeitungseinheit 48 und die Datenspeichereinheit 50 stellen somit Mittel 56 zur Ausführung eines Verfahrens zum Überwachen einer blutbezogenen, individuellen anaeroben Schwelle ANS eines Probanden 10 dar.

Im Folgenden wird das Verfahren zum Überwachen einer blutbezogenen, individuellen anaeroben Schwelle ANS näher erläutert.

In einem ersten Schritt S21 dieses Verfahrens werden zweite Daten D2 erhalten, welche Speichellaktatkonzentrationswerte KS und einen jeweils zugehörigen Erfassungszeitpunkt beschreibende Zeitwerte T umfassen. Diese zweiten Daten D2 werden von der ersten Sensoreinheit 40 und von der Zeiteinheit 44 bereitgestellt.

Darüber hinaus wird in einem zweiten Schritt S22, der vor, während oder nach dem ersten Schritt S21 ausgeführt werden kann, die blutbezogene, individuelle anaerobe Schwelle ANS des Probanden 10 erhalten. Diese kann in Form von dritten Daten D3 vorliegen.

Dabei kann die blutbezogene, individuelle anaeroben Schwelle ANS des Probanden 10 mittels des zuvor erläuterten Verfahrens zum Bestimmen der blutbezogenen, individuellen anaeroben Schwelle ANS ermittelt worden sein.

Alternativ hierzu kann die blutbezogene, individuelle anaerobe Schwelle ANS durch Erhalten einer zeitlichen Änderungsrate der Speichellaktatkonzentrationswerte KS der zweiten Daten D2 und Erhalten einer speichelbezogenen, individuellen anaeroben Schwelle SANS basierend auf der zeitlichen Änderungsrate der Speichellaktatkonzentrationswerte KS ermittelt werden. Dabei muss ferner die speichelbezogene, individuelle anaerobe Schwelle SANS in die blutbezogene, individuelle anaerobe Schwelle ANS umgerechnet werden. Dies wurde weiter oben bereits im Zusammenhang mit dem Verfahren zum Bestimmen der blutbezogenen, individuellen anaeroben Schwelle ANS erläutert.

Es wird betont, dass in beiden Varianten die blutbezogene, individuelle anaerobe Schwelle ANS bereitgestellt werden kann, ohne dass hierfür Blutentnahmen und/oder Blutanalysen notwendig sind.

In einem dritten Schritt S23 des Verfahrens zum Überwachen der blutbezogenen, individuellen anaeroben Schwelle ANS wird ein Indikator bereitgestellt, welcher eine Beziehung der zweiten Daten D2 zur speichelbezogenen, individuellen anaeroben Schwelle SANS oder zur blutbezogenen, individuellen anaeroben Schwelle ANS beschreibt.

Dies kann grafisch unter Nutzung der als Bildschirmeinheit ausgebildeten Ausgabeschnittstelle 46 erfolgen.

Mit Bezug auf die speichelbezogene, individuelle anaerobe Schwelle SANS kann dem Probanden 10 also über die Bildschirmeinheit ausgegeben werden, ob die in den zweiten Daten D2 enthaltenen Speichellaktatkonzentrationswerte KS auf eine Leistung unterhalb, oberhalb oder auf der speichelbezogenen, individuellen anaeroben Schwelle SANS hindeuten.

Der Proband 10 kann diese Information nutzen, um sein Training zu steuern. Aufgrund der Tatsache, dass die Speichellaktatkonzentration KS der Blutlaktatkonzentration KB zeitlich üblicherweise um 6 bis 10 Minuten nachläuft, ist dies insbesondere dann nützlich, wenn die Belastung, d.h. die vom Probanden abgegebene Leistung P, über den Belastungszeitraum wenig schwankt und der Belastungszeitraum vergleichsweise lang ist.

Alternativ oder zusätzlich kann der Indikator, welcher eine Beziehung der zweiten Daten D2 zur speichelbezogenen, individuellen anaeroben Schwelle SANS beschreibt, über die Trainingsdauer und eine gewisse Zeit darüber hinaus aufgezeichnet werden, sodass das Training dokumentiert werden kann. Dabei werden insbesondere Zeitspannen und/oder Zeitanteile dokumentiert, während denen die in den zweiten Daten D2 enthaltenen Speichellaktatkonzentrationswerte KS auf eine Leistung unterhalb, oberhalb oder auf der speichelbezogenen, individuellen anaeroben Schwelle SANS hindeuten.

Mit Bezug auf die blutbezogene, individuelle anaerobe Schwelle ANS kann dem Probanden über die Bildschirmeinheit ausgegeben werden, ob die in den zweiten Daten D2 enthaltenen Speichellaktatkonzentrationswerte KS auf eine Leistung unterhalb, oberhalb oder auf der blutbezogenen, individuellen anaeroben Schwelle ANS hindeuten.

Der Proband 10 kann diese Beziehung der zweiten Daten D2 zur speichelbezogenen, individuellen anaeroben Schwelle SANS oder zur blutbezogenen, individuellen anaeroben Schwelle ANS verwenden, um sein Training zu steuern und somit effizienter und/oder wirkungsvoller zu gestalten.

Optional ist das System 38 zum Überwachen seiner blutbezogenen, individuellen anaeroben Schwelle ANS ferner dazu ausgebildet, Trainingsbereiche anzugeben, die anhand der blutbezogenen, individuellen aeroben Schwelle AES des Probanden 10 und der blutbezogenen, individuellen anaeroben Schwelle ANS des Probanden 10 definiert sind.

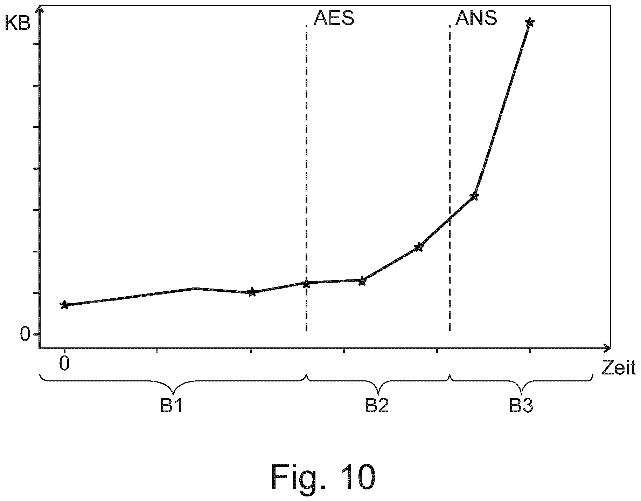

In diesem Zusammenhang ist ein erster Trainingsbereich B1 dadurch charakterisiert ist, dass ein Blutlaktatkonzentrationswert KB unterhalb der blutbezogenen, individuellen aeroben Schwelle AES des Probanden 10 liegt (siehe Figur 10).

Ein zweiter Trainingsbereich B2 ist dadurch charakterisiert, dass ein Blutlaktatkonzentrationswert KB zwischen der blutbezogenen, individuellen aeroben Schwelle AES des Probanden 10 und der blutbezogenen, individuellen anaeroben Schwelle ANS des Probanden 10 liegt.

Ein dritter Trainingsbereich B3 ist dadurch charakterisiert, dass ein Blutlaktatkonzentrationswert KB oberhalb der blutbezogenen, individuellen anaeroben Schwelle ANS des Probanden 10 liegt.

Zur Bestimmung dieser Trainingsbereiche B1, B2, B3 können die blutbezogene, individuelle aerobe Schwelle AES des Probanden 10 und die blutbezogene, individuelle anaeroben Schwelle ANS des Probanden 10 in gleicher Weise bestimmt werden, wie oben bereits erläutert.

Alternativ ist es möglich, die Trainingsbereiche B1, B2, B3 basierend auf den zweiten Daten D2 und unter Nutzung eines weiteren Datenmodells, insbesondere ein entsprechend trainiertes Datenmodell, direkt zu bestimmen. In einem solchen Fall müssen zuvor die blutbezogene, individuelle aerobe Schwelle AES des Probanden 10 und die blutbezogene, individuelle anaeroben Schwelle ANS des Probanden 10 nicht explizit bestimmt werden.

Die Trainingsbereiche können ebenfalls zur Trainingssteuerung und/oder zur Trainingsdokumentation genutzt werden. Um dem Probanden 10 einen aktuellen Trainingsbereich B1, B2, B3 und/oder Trainingsbereiche B1, B2, B3 aus der Vergangenheit anzuzeigen, kann wieder die Ausgabeschnittstelle 46 des Systems 38 zum Überwachen einer blutbezogenen, individuellen anaeroben Schwelle ANS genutzt werden.

Es versteht sich, dass aufgrund der Tatsache, dass die Trainingsbereiche B1, B2, B3 auch ohne explizite Angabe der blutbezogenen, individuellen aeroben Schwelle AES des Probanden 10 und der blutbezogenen, individuellen anaeroben Schwelle ANS des Probanden 10 bestimmt werden können, die Trainingsbereiche B1, B2, B3 auch unabhängig vom Verfahren zum Überwachen einer blutbezogenen, individuellen anaeroben Schwelle ANS des Probanden 10, d.h. eigenständig, bestimmt werden können.

Vorliegend wurden das System 16 und das Verfahren zum Bestimmen einer blutbezogenen, individuellen anaeroben Schwelle ANS anhand eines Probanden 10 erläutert, der einen Fahrradergometer 12 nutzt. Das System 38 und Verfahren zum Überwachen der blutbezogenen, individuellen anaeroben Schwelle ANS wurde anhand eines Probanden 10 erläutert, der auf einem Fahrrad 36 fährt. Es versteht sich dabei, dass diese Sportart im Sinne einer einfachen Erläuterung gewählt wurde. Selbstverständlich ist es auch möglich, die oben beschriebenen Systeme 16, 38 und Verfahren im Zusammenhang mit anderen Sportarten zu verwenden.

Darüber hinaus versteht es sich, dass auch wenn vorliegend derselbe Proband 10 zur Erläuterung des Systems 16 und des Verfahrens zum Bestimmen einer blutbezogenen, individuellen anaeroben Schwelle ANS und zur Erläuterung des Systems 38 und Verfahrens zum Überwachen der blutbezogenen, individuellen anaeroben Schwelle ANS herangezogen wurde, es selbstverständlich auch möglich ist, hier unterschiedliche Probanden zu verwenden, d. h. dass ein Proband das System 16 und Verfahren zum Bestimmen einer blutbezogenen, individuellen anaeroben Schwelle ANS nutzt und ein anderer Proband das System 38 und Verfahren zur Überwachung der blutbezogenen, individuellen anaeroben Schwelle ANS.

Darüber hinaus wurden vorliegend die Vorrichtung 22 zur Datenverarbeitung und deren Komponenten als von der Vorrichtung 42 zur Datenverarbeitung und deren Komponenten separate Einheiten präsentiert. Das ist eine Möglichkeit. Es ist jedoch auch möglich, dass eine einzige Vorrichtung zur Datenverarbeitung dazu ausgebildet ist, sowohl das Verfahren zum Bestimmen der blutbezogenen, individuellen anaeroben Schwelle ANS als auch das Verfahren zum Überwachen der blutbezogenen, individuellen anaeroben Schwelle ANS auszuführen.

Eine derartige Vorrichtung zur Datenverarbeitung kann also sowohl Bestandteil eines Systems 16 zum Bestimmen der blutbezogenen, individuellen anaeroben Schwelle ANS als auch Bestandteil eines Systems 38 zum Überwachen der blutbezogenen, individuellen anaeroben Schwelle ANS sein.

### Bezugszeichenliste

- 10: Proband
- 12: Fahrradergometer
- 14: Pedale
- 16: System zum Bestimmen einer blutbezogenen, individuellen anaeroben Schwelle
- 18: erste Sensoreinheit zum Erfassen wenigstens eines Speichellaktatkonzentrationswerts
- 20: zweite Sensoreinheit zum Erfassen wenigstens eines Leistungswerts
- 22: Vorrichtung zur Datenverarbeitung
- 24: Zeiteinheit
- 26: Datenverarbeitungseinheit
- 28: Datenspeichereinheit
- 30: computerlesbares Medium
- 32: Computerprogramm
- 34: Mittel zur Ausführung des Verfahrens zum Bestimmen einer blutbezogenen, individuellen anaeroben Schwelle eines Probanden
- 36: Fahrrad
- 38: System zum Überwachen einer blutbezogenen, individuellen anaeroben Schwelle
- 40: erste Sensoreinheit
- 42: Vorrichtung zur Datenverarbeitung
- 44: Zeiteinheit
- 46: Ausgabeschnittstelle
- 48: Datenverarbeitungseinheit
- 50: Datenspeichereinheit
- 52: computerlesbares Medium
- 54: Computerprogramm
- 56: Mittel zur Ausführung des Verfahrens zum Überwachen einer blutbezogenen, individuellen anaeroben Schwelle eines Probanden
- ANS: blutbezogene, individuelle anaerobe Schwelle
- AES: blutbezogene, individuelle aerobe Schwelle
- B1: erster Trainingsbereich
- B2: zweiter Trainingsbereich
- B3: dritter Trainingsbereich
- D1: erste Daten
- D2: zweite Daten
- D3: dritte Daten
- DT: Trainingsdatenelement
- KS: Speichellaktatkonzentrationswert
- KB: Blutlaktatkonzentrationswert
- M: Datenmodell
- P: Leistungswert
- SANS: speichelbezogene, individuelle anaerobe Schwelle
- SAES: speichelbezogene, individuelle aerobe Schwelle
- S11: erster Schritt des Verfahrens zum Bestimmen einer blutbezogenen, individuellen anaeroben Schwelle
- S12: zweiter Schritt des Verfahrens zum Bestimmen einer blutbezogenen, individuellen anaeroben Schwelle
- S13: dritter Schritt des Verfahrens zum Bestimmen einer blutbezogenen, individuellen anaeroben Schwelle
- S14: vierter Schritt des Verfahrens zum Bestimmen einer blutbezogenen, individuellen anaeroben Schwelle
- S21: erster Schritt des Verfahrens zum Überwachen einer blutbezogenen, individuellen anaeroben Schwelle
- S22: zweiter Schritt des Verfahrens zum Überwachen einer blutbezogenen, individuellen anaeroben Schwelle
- S23: dritter Schritt des Verfahrens zum Überwachen einer blutbezogenen, individuellen anaeroben Schwelle
- T: Zeitwert

## Patentansprüche

1. Verfahren zum Bestimmen einer blutbezogenen, individuellen anaeroben Schwelle (ANS) eines Probanden (10), umfassend:
- Erhalten von ersten Daten (D1), welche eine Leistung des Probanden (10) beschreibende Leistungswerte (P), einen jeweils zugehörigen Erfassungszeitpunkt beschreibende Zeitwerte (T) und jeweils zugehörige Speichellaktatkonzentrationswerte (KS) umfassen, wobei die ersten Daten (D1) über einen maximalen Speichellaktatkonzentrationswert (KS) hinausgehen (S11), und
- Erhalten der blutbezogenen, individuellen anaeroben Schwelle (ANS) basierend auf den ersten Daten (D1) und unter Nutzung eines Datenmodells (M) (S14).

2. Verfahren nach Anspruch 1, wobei das Datenmodell (M) ein trainiertes Datenmodell (M) umfasst, welches die ersten Daten (D1) als Eingangsdaten verwendet und dazu ausgebildet ist, die blutbezogene, individuelle anaerobe Schwelle (ANS) auszugeben.

3. Verfahren nach Anspruch 1 oder 2,
wobei das Datenmodell (M) ein Regressionsmodell umfasst, welches einen Zusammenhang zwischen der blutbezogenen, individuellen anaeroben Schwelle (ANS) und einer speichelbezogenen, individuellen anaeroben Schwelle (SANS) beschreibt, und/oder
wobei das Datenmodell (M) ein Regressionsmodell umfasst, welches einen Zusammenhang zwischen der blutbezogenen, individuellen aeroben Schwelle (AES) und einer speichelbezogenen, individuellen aeroben Schwelle (SAES) beschreibt, und das Datenmodell (M) zusätzlich ein Regressionsmodell umfasst, welches einen Zusammenhang zwischen einem maximalen Speichellaktatkonzentrationswert (KS) und einem maximalen Blutlaktatkonzentrationswert (KB) beschreibt.

4. Verfahren nach einem der vorhergehenden Ansprüche, wobei das Erhalten der blutbezogenen, individuellen anaeroben Schwelle (ANS) ein Erhalten einer speichelbezogenen, individuellen anaeroben Schwelle (SANS) basierend auf den ersten Daten (D1) umfasst (S13).

5. Verfahren nach Anspruch 4, wobei das Erhalten der speichelbezogenen, individuellen anaeroben Schwelle (SANS) ein Erhalten einer speichelbezogenen, individuellen aeroben Schwelle (SAES) umfasst (S12).

6. Verfahren nach Anspruch 5, wobei die speichelbezogene, individuelle aerobe Schwelle (SAES) als derjenige Leistungswert (P) erhalten wird, bei dem ein Quotient aus Speichellaktatkonzentrationswert (KS) und zugehörigem Leistungswert (P) minimal ist oder wobei die speichelbezogene, individuelle aerobe Schwelle (SAES) ein Auswerten einer zeitlichen Änderungsrate der Speichellaktatkonzentrationswerte (KS) umfasst.

7. Verfahren nach einem der Ansprüche 4 bis 6, wobei das Erhalten der speichelbezogenen, individuellen anaeroben Schwelle (SANS) ein Ermitteln eines maximalen Abstands einer Geraden, welche sich von einem minimalen Speichellaktatkonzentrationswert (KS) zum maximalen Speichellaktatkonzentrationswert (KS) erstreckt oder welche sich von einem der individuellen aeroben Schwelle (SAES) zugeordneten Speichellaktatkonzentrationswert (KS) zum maximalen Speichellaktatkonzentrationswert (KS) erstreckt, von einer die Speichellaktatkonzentrationswerte (KS) verbindenden Kurve umfasst,
wobei die speichelbezogene, individuelle anaerobe Schwelle (SANS) als Leistungswert (P) am Punkt des maximalen Abstands erhalten wird.

8. Verfahren nach Anspruch 4, wobei das Erhalten der speichelbezogenen, individuellen anaeroben Schwelle (SANS) ein Auswerten einer zeitlichen Änderungsrate der Speichellaktatkonzentrationswerte (KS) umfasst.

9. Verfahren zum Überwachen einer blutbezogenen, individuellen anaeroben Schwelle (ANS) eines Probanden (10), umfassend:
- Erhalten von zweiten Daten (D2), welche Speichellaktatkonzentrationswerte (KS) und einen jeweils zugehörigen Erfassungszeitpunkt beschreibende Zeitwerte (T) umfassen (S21),
- Erhalten der blutbezogenen, individuellen anaeroben Schwelle (ANS) des Probanden (10), wobei die blutbezogene, individuelle anaerobe Schwelle (ANS) des Probanden (10) mittels des Verfahrens nach einem der vorhergehenden Ansprüche ermittelt ist (S22), oder
Erhalten einer zeitlichen Änderungsrate der Speichellaktatkonzentrationswerte (KS) und Erhalten einer speichelbezogenen, individuellen anaeroben Schwelle (SANS) basierend auf der zeitlichen Änderungsrate der Speichellaktatkonzentrationswerte (KS), und
- Bereitstellen eines Indikators, welcher eine Beziehung der zweiten Daten (D2) zur speichelbezogenen, individuellen anaeroben Schwelle (SANS) oder zur blutbezogenen, individuellen anaeroben Schwelle (ANS) beschreibt (S23).

10. Verfahren zum Trainieren eines Datenmodells (M) zum Bestimmen einer blutbezogenen, individuellen anaeroben Schwelle (ANS), wobei das Datenmodell (M) mittels eines annotierten Datensatzes trainiert wird, welcher mehrere Trainingsdatenelemente (DT) umfasst, welche eine Leistung beschreibende Leistungswerte (P), einen jeweils zugehörigen Erfassungszeitpunkt beschreibende Zeitwerte (T) und jeweils zugehörige Speichellaktatkonzentrationswerte (KS) umfassen, wobei die Trainingsdatenelemente (DT) mit zugehörigen blutbezogenen, individuellen anaeroben Schwellen (ANS) annotiert sind.

11. Vorrichtung (22, 42) zur Datenverarbeitung, umfassend Mittel (34, 56) zur Ausführung wenigstens eines der Verfahren nach einem der Ansprüche 1 bis 10.

12. Computerprogramm (32, 54), umfassend Befehle, die bei der Ausführung des Computerprogramms (32, 54) durch einen Computer diesen veranlassen, wenigstens eines der Verfahren nach einem der Ansprüche 1 bis 10 auszuführen.

13. Computerlesbares Medium (30, 52), umfassend Befehle, die bei der Ausführung durch einen Computer diesen veranlassen, wenigstens eines der Verfahren nach einem der Ansprüche 1 bis 10 auszuführen.

14. System (16) zum Bestimmen einer blutbezogenen, individuellen anaeroben Schwelle (ANS) eines Probanden (10), umfassend:
- eine Vorrichtung (22) zur Datenverarbeitung, umfassend Mittel (34) zur Ausführung des Verfahrens nach einem der Ansprüche 1 bis 8,
- eine erste Sensoreinheit (18) zum Erfassen wenigstens eines Speichellaktatkonzentrationswerts (KS),
- eine zweite Sensoreinheit (20) zum Erfassen wenigstens eines Leistungswerts (P), welcher eine Leistung des Probanden (10) beschreibt,
- eine Zeiteinheit (24) zum Bereitstellen eines Zeitwerts (T),
wobei die erste Sensoreinheit (18), die zweite Sensoreinheit (20) und die Zeiteinheit (24) kommunikationstechnisch mit der Vorrichtung (22) zur Datenverarbeitung gekoppelt sind oder Bestandteile der Vorrichtung (22) zur Datenverarbeitung sind.

15. System (38) zum Überwachen einer blutbezogenen, individuellen anaeroben Schwelle (ANS) eines Probanden (10), umfassend:
- eine Vorrichtung (42) zur Datenverarbeitung, umfassend Mittel (56) zur Ausführung des Verfahrens nach Anspruch 9,
- eine erste Sensoreinheit (40) zum Erfassen wenigstens eines Speichellaktatkonzentrationswerts (KS),
- eine Zeiteinheit (44) zum Bereitstellen eines Zeitwerts (T),
- eine Ausgabeschnittstelle (46) zum Ausgeben eines Indikators, welcher eine Beziehung wenigstens eines Speichellaktatkonzentrationswerts (KS), der zu einem zugehörigen Erfassungszeitpunkt (T) erfasst wurde, zur speichelbezogenen, individuellen anaeroben Schwelle (SANS) oder zur blutbezogenen, individuellen anaeroben Schwelle (ANS) beschreibt,
wobei die erste Sensoreinheit (40), die Zeiteinheit (44) und die Ausgabeschnittstelle (46) kommunikationstechnisch mit der Vorrichtung (42) zur Datenverarbeitung gekoppelt sind oder Bestandteile der Vorrichtung (42) zur Datenverarbeitung sind.
